# EUROPEAN PATENT APPLICATION

(11) **EP 4 428 156 A1**
(43) Date of publication of application: **11.09.2024**
(21) Application number: 22900650.7
(22) Date of filing: 02.12.2022
(51) Int. Cl.: C07K 16/28, A61P 35/00, A61K 39/395, C12N 15/13

(54) **ANTI-BCMA NANOBODY AND USE THEREOF**

(30) Priority: 03.12.2021 CN 202111468274; 19.08.2022 CN 202211000054
(71) Applicant: Shandong Simcere Biopharmaceutical Co., Ltd., Shandong 264006 (CN)
(72) Inventor: FU, Yayuan, Shanghai 201318 (CN); YOU, Shumei, Shanghai 201318 (CN); YIN, Bowei, Shanghai 201318 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: Bandpay & Greuter
(86) International application number: PCT/CN2022/136074
(87) International publication number: WO 2023/098846

(57) **Abstract**

An antibody, or an antigen-binding fragment of same, specifically binding to a BCMA, a nucleic acid encoding same, an expression vector and an expression cell thereof, a preparation method therefor, a pharmaceutical composition thereof, and the use thereof in treating a disease, for example, the use thereof in treating a tumour.

## Description

### Cross Reference to Related Patent Application

The present application claims the priority of China patent application with patent application number 202111468274.X filed with China National Intellectual Property Administration on December 03, 2021, and with the invention title of BCMA NANOBODY AND USE THEREOF, and the China patent application with the patent application number of 20221100054.9 filed with China National Intellectual Property Administration on August 19, 2022 and with the invention title of ANTI-BCMA NANOBODY AND USE THEREOF. The above-mentioned prior applications are incorporated into the present application by reference in their entirety.

### Technical Field

The present application relates to the field of biomedicine, and specifically to an anti-BCMA nanobody and the use thereof.

### Background

Multiple myeloma (MM) is a malignant plasma cell tumor originating in the bone marrow, is a type of B-cell lymphoma and is also called plasma cell tumor. It is characterized by abnormal proliferation of bone marrow plasma cells accompanied by overproduction of monoclonal immunoglobulin or light chain (M protein). A very small number of patients may have unsecreted MM that does not produce M protein. Multiple myeloma is often accompanied by multiple osteolytic lesions, hypercalcemia, anemia, and renal damage. Since the production of normal immunoglobulins is inhibited, various bacterial infections are prone to occur.

Multiple myeloma accounts for 1% of all tumors and 10%-15% of hematologic malignancies. The ratio of male to female is 1.6 : 1, and most patients are over 40 years old. The treatment of multiple myeloma includes chemotherapy, hematopoietic stem cell transplantation, *etc.* Among them, immunomodulators represented by lenalidomide and protease inhibitors represented by bortezomib, used either alone or in combination, have shown good efficacy, and have become conventional treatment methods for patients with multiple myeloma. However, multiple myeloma is still considered as an incurable disease. At present, treatment methods can only relieve the symptoms of multiple myeloma, but none of them can completely eliminate the tumor. Almost all patients will eventually relapse. Therefore, there is an urgent need for new treatment options.

B cell maturation antigen (BCMA), also known as CD269 or TNFRSF17, is a member of the tumor necrosis factor receptor superfamily and was first identified in the early 1990s. This receptor is mainly expressed on the surface of mature B lymphocytes and plasma cells, is a marker protein of B lymphocyte maturation and is hardly expressed in other tissue cells. Structurally, BCMA consists of three main domains: an extracellular segment (aa1-54), a transmembrane region (aa55-77) and an intracellular segment (aa78-184). B cell activating factor (BAFF) and a proliferation inducing ligand (APRIL) are the main ligands of BCMA, which transmit cell stimulation signals by interacting with BCMA, activate TRAF-dependent NF-κB and JNK pathways and increase the proliferation and survival rate of B cells. BCMA is highly expressed in MM cells and is an ideal antigen target for multiple myeloma. The homology of BCMA between human and monkeys is 88%, so it is difficult to screen cross-binding antibodies.

Treatments such as CAR T, bispecific antibodies and ADCs targeting BCMA have made important progress and show bright prospects. However, there is still an urgent need to develop a new generation of more efficient BCMA-specific antibodies and biotherapeutic products based on same.

### Summary of the Invention

The present application provides an antibody or an antigen-binding fragment thereof that specifically binds to BCMA, a multispecific antigen-binding molecule, a chimeric antigen receptor, an immune effector cell, a nucleic acid fragment, a vector, a host cell, a pharmaceutical composition, a kit, a preparation method, and the use of the antibody or the antigen-binding fragment thereof in the treatment of diseases and detection of BCMA.

In one aspect, the present application provides an antibody or an antigen-binding fragment thereof that specifically binds to BCMA, wherein the antibody or the antigen-binding fragment thereof comprises CDR1, CDR2 and CDR3, the CDR1 comprises HCDR1 of VHH domain shown in any sequence of SEQ ID NO: 7-21, 24-31 and 92-101, the CDR2 comprises HCDR2 of VHH domain shown in any sequence of SEQ ID NO: 7-21, 24-31 and 92-101,and the CDR3 comprises HCDR3 of VHH domain shown in any sequence of SEQ ID NO: 7-21, 24-31 and 92-101.

In another aspect, the present application provides a multispecific antigen-binding molecule, wherein the multispecific antigen-binding molecule comprises the aforementioned antibody or the antigen-binding fragment thereof, and an antigen-binding molecule binding to an antigen other than BCMA or binding to a different BCMA epitope than that of the aforementioned antibody or the antigen-binding fragment thereof.

In another aspect, the present application provides an isolated nucleic acid fragment, wherein the nucleic acid fragment encodes the aforementioned antibody or the antigen-binding fragment thereof or the aforementioned multispecific antigen-binding molecule.

In another aspect, the present application provides a vector, wherein the vector comprises the aforementioned nucleic acid fragment.

In another aspect, the present application provides a host cell, wherein the host cell comprises the aforementioned nucleic acid fragment or the aforementioned vector.

In another aspect, the present application provides a method for preparing the aforementioned antibody or the antigen-binding fragment thereof or the aforementioned multispecific antigen-binding molecule, wherein the method comprises culturing the aforementioned cell, and isolating the antibody, antigen-binding fragment or multispecific antigen-binding molecule expressed by the cell.

In another aspect, the present application provides a pharmaceutical composition, wherein the pharmaceutical composition comprises the aforementioned antibody or the antigen-binding fragment thereof, the aforementioned multispecific antigen-binding molecule, the aforementioned nucleic acid fragment, the aforementioned vector, or a product prepared according to the aforementioned method.

In another aspect, the present application provides a method for treating tumors or cancers, wherein the method comprises administering to a subject an effective amount of the aforementioned antibody or the antigen-binding fragment thereof, the aforementioned multispecific antigen-binding molecule, the aforementioned nucleic acid fragment, the aforementioned vector, a product prepared according to the aforementioned method or the aforementioned pharmaceutical composition.

In another aspect, the present application provides the use of the aforementioned antibody or the antigen-binding fragment thereof, the aforementioned multispecific antigen-binding molecule, the aforementioned nucleic acid fragment, the aforementioned vector, a product prepared according to the aforementioned method or the aforementioned pharmaceutical composition in preparing a drug for treating a tumor or a cancer.

In another aspect, the present application provides the use of the aforementioned antibody or the antigen-binding fragment thereof, the aforementioned multispecific antigen-binding molecule, the aforementioned nucleic acid fragment, the aforementioned vector, a product prepared according to the aforementioned method or the aforementioned pharmaceutical composition for treating a tumor or a cancer.

In another aspect, the present application provides a kit, wherein the kit comprises the aforementioned antibody or the antigen-binding fragment thereof, the aforementioned multispecific antigen-binding molecule, the aforementioned nucleic acid fragment, the aforementioned vector, a product prepared according to the aforementioned method or the aforementioned pharmaceutical composition.

In another aspect, the present application provides a method for detecting BCMA expression in a biological sample, wherein the method comprises contacting the biological sample with the aforementioned antibody or the antigen-binding fragment thereof under conditions that allow the formation of a complex from the antibody or the antigen-binding fragment thereof and BCMA.

In another aspect, the present application provides the use of the aforementioned antibody or the antigen-binding fragment thereof in preparing a BCMA detection reagent.

This application provides an antibody or an antigen-binding fragment thereof with higher affinity for the BCMA target, which can better block the binding of BCMA to its ligand APRIL, thereby providing a better choice for BCMA antibody drugs and cell therapy products. It is of great significance to fill the gap in the treatment of multiple myeloma, especially relapsed/refractory multiple myeloma.

### Brief Description of the Figures

Fig. 1A shows the ELISA detection of the binding activity of control antibody with human BCMA-His protein;
Fig. 1B shows the binding activity of control antibody with monkey BCMA-His protein detected by ELISA;
Fig. 2A shows the FACS results of detecting BCMA expression in H929 cells using REGN5459-hIgG1 and HPN217-hHcAb antibodies;
Fig. 2B shows the FACS results of detecting BCMA expression in U266 cells using REGN5459-hIgG1 and HPN217-hHcAb antibodies;
Fig. 3 shows the FACS results of detecting BCMA expression in Flp-inCHO-human BCMA cells using REGN5459-hIgG1 antibody;
Fig. 4 shows the FACS results of detecting BCMA expression in Flp-inCHO-monkey BCMA cells using REGN5459-hIgG1 antibody;
Fig. 5 shows the ELISA detection of the binding activity of recombinant candidate antibodies with human BCMA-his protein;
Fig. 6 shows the ELISA detection of the binding activity of recombinant candidate antibodies with monkey BCMA-his protein;
Fig. 7 shows the FACS detection of the binding activity of recombinant candidate antibodies with H929 tumor cells;
Fig. 8 shows the FACS detection of the binding activity of recombinant candidate antibodies with U266 tumor cells;
Fig. 9 shows the ligand binding competition ELISA to detect the blocking effect of recombinant candidate antibodies on the binding of ligand APRIL to human BCMA protein;
Fig. 10 shows the ELISA detection of the binding activity of humanized candidate nanobodies with human BCMA-his protein;
Fig. 11 shows the ELISA detection of the binding activity of humanized candidate nanobodies with monkey BCMA-his protein;
Fig. 12 shows the FACS detection of the binding activity of humanized candidate nanobodies with H929 tumor cells;
Fig. 13 shows the FACS detection of the binding activity of humanized candidate nanobodies with U266 tumor cells;
Fig. 14 shows the ligand binding competition ELISA to detect the blocking effect of humanized candidate nanobodies on the binding of ligand APRIL to human BCMA protein;
Fig. 15 shows ELISA detection of the binding activity of chimeric antibody with human BCMA-his protein;
Fig. 16A shows the FACS detection of the binding activity of chimeric antibodies with H929 tumor cells;
Fig. 16B shows the FACS detection of the binding activity of chimeric antibodies with U266 tumor cells;
Fig. 17 shows the ligand binding competition ELISA to detect the blocking effect of chimeric antibodies on the binding of ligand APRIL to human BCMA protein.

### Detailed Description of the Invention

### Definition and Description of Terminology

Unless otherwise defined herein, scientific and technical terms related to the present application shall have the meanings understood by one of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of'.

The term "and/or" as used herein includes the meanings of "and", "or" and "all or any other combination of elements linked by the term".

The term "BCMA" herein is the abbreviated name of B cell maturation antigen, which is a member of the tumor necrosis factor receptor family. BCMA is mainly expressed on the surface of late B cells, short-lived proliferative plasma cells and long-lived plasma cells, and is not expressed in naive B cells, CD34-positive hematopoietic stem cells and other normal tissue cells, but is highly expressed in MM cells. BCMA plays a key role in the survival, proliferation, metastasis and drug resistance of MM cells by mediating downstream signaling pathways, thereby being an ideal target antigen for the treatment of MM.

The term "specifically bind" herein means that an antigen-binding molecule (e.g., an antibody) typically specifically binds to an antigen and substantially the same antigen with a high affinity, but does not bind to an unrelated antigen with a high affinity. Affinity is generally reflected by the equilibrium dissociation constant (KD), where lower KD indicates higher affinity. Taking an antibody as an example, a high affinity generally means having a KD of about 10⁻⁶ M or less, about 10⁻⁷ M or less, about 10⁻⁸ M or less or about 10⁻⁹ M or less. KD is calculated as follows: KD = Kd/Ka, where Kd represents the dissociation rate and Ka represents the binding rate. The equilibrium dissociation constant KD can be measured using well-known methods in the art, such as surface plasmon resonance (e.g., Biacore) or equilibrium dialysis assay.

The term "antigen-binding molecule" is used herein in the broadest sense and refers to a molecule specifically binding to an antigen. Exemplarily, the antigen-binding molecule includes, but is not limited to, an antibody or an antibody mimetic. The "antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. The "antibody" herein encompasses various forms and various structures as long as they exhibit the desired antigen-binding activity. The "antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, e.g., Korndorfer et al., 2003, Proteins: Structure, Function, and Bioinformatics, 53(1): 121-129 (2003); Roque et al., Biotechnol. Prog. 20: 639-654 (2004). Such scaffolds may also include non-antibody-derived scaffolds, for example, scaffold proteins known in the art which can be used for grafting CDRs, including but not limited to tenascin, fibronectin, a peptide aptamer, etc.

The term "antibody" includes an intact antibody and any antigen-binding fragment (i.e., "antigen-binding moiety") or single chain thereof. The "antibody" refers to a glycoprotein comprising at least two heavy (H) chains and two light (L) chains interconnected by a disulfide bond, or an antigen-binding moiety thereof. Each heavy chain consists of a heavy chain variable region (abbreviated herein as VH) and a heavy chain constant region. The heavy chain constant region consists of three domains, CH1, CH2 and CH3. Each light chain consists of a light chain variable region (abbreviated herein as VL) and a light chain constant region. The light chain constant region consists of one domain, CL. The VH and VL regions can be further subdivided into hypervariable regions known as complementarity determining regions (CDRs), which are interspersed among more conserved regions known as framework regions (FRs). Each VH and VL consists of three CDRs and four FRs, which are arranged in the following order from amino terminus to carboxyl terminus: FR1, CDR1, FR2, CDR2, FR3, CDR3, FR4. The variable regions of the heavy and light chains contain a binding domain that can interact with an antigen. The constant region of an antibody can mediate the binding of an immunoglobulin to a host tissue or factor, and the host tissue or factor includes various cells of the immune system (e.g., effector cells) and a first component of the classical complement system (C1q). Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

"Antibody" herein also includes an antibody that does not comprise light chains, for example, heavy-chain antibodies (HCAbs) produced from camelids, such as Camelus dromedarius, Camelus bactrianus, Lama glama, Lama guanicoe and Vicugna pacos, and Ig new antigen receptors (IgNARs) found in Chondrichthyes such as sharks.

The term "antibody" herein may be derived from any animal, including but not limited to humans and non-human animals selected from primates, mammals, rodents and vertebrates, such as Camelidae, Lama glama, Lama guanicoe, Vicugna pacos, sheep, rabbits, mice, rats or Chondrichthyes (such as sharks).

The term "heavy chain antibody" herein refers to an antibody that lacks light chains of a conventional antibody. The term specifically includes, but is not limited to, a homodimeric antibody comprising a VH antigen-binding domain and CH2 and CH3 constant domains in the absence of a CH1 domain.

The term "nanobody" herein refers to a single domain antibody, also known as VHH (Variable domain of heavy chain of heavy chain antibody) obtained by cloning the variable region of heavy chain antibody (a natural heavy chain antibody without light chains in camel, *etc*.), which only consists of a heavy chain variable region, and is the smallest functional antigen-binding fragment.

The terms "VHH domain", "nanobody" and "single domain antibody" (sdAb) herein have the same meaning and are used interchangeably, and refer to a single domain antibody consisting of only one heavy chain variable region constructed by cloning the variable region of a heavy chain antibody, which is the smallest antigen-binding fragment with full functionality. Generally, a single domain antibody consisting of only one heavy chain variable region is constructed by obtaining a heavy chain antibody that naturally lacks light chains and heavy chain constant region 1 (CH1), and then cloning the heavy chain variable region of the antibody.

Further descriptions of "heavy chain antibody" and "single domain antibody", and "VHH domain" and "nanobody" can be found in Hamers-Casterman et al., Nature. 1993; 363; 446-8; Muyldermans' review article (Reviews in Molecular Biotechnology 74: 277-302, 2001); and the following patent applications mentioned as general background art: WO 94/04678, WO 95/04079 and WO 96/34103; WO 94/25591, WO 99/37681, WO 00/40968, WO 00/43507, WO 00/65057, WO 01/40310, WO 01/44301, EP 1134231 and WO 02/48193; WO 97/49805, WO 01/21817, WO 03/035694, WO 03/054016 and WO 03/055527; WO 03/050531; WO 01/90190; WO 03/025020; and WO 04/041867, WO 04/041862, WO 04/041865, WO 04/041863, WO 04/062551, WO 05/044858, WO 06/40153, WO 06/079372, WO 06/122786, WO 06/122787 and WO 06/122825, and other prior art mentioned in these applications.

The term "multispecific" herein refers to the ability of an antibody or an antigen-binding fragment thereof to bind to, for example, different antigens or at least two different epitopes on the same antigen. Therefore, the terms such as "bispecific", "trispecific" and "tetraspecific" refer to the number of different epitopes to which an antibody can bind. For example, a conventional monospecific IgG-type antibody has two identical antigen-binding sites (paratopes) and thus can only bind to the same epitope (rather than bind to different epitopes). In contrast, a multispecific antibody has at least two different types of paratopes/binding sites and thus can bind to at least two different epitopes. As described herein, "complementarity determining region" refers to an antigen-binding site of an antibody. Furthermore, single "specific" may refer to one, two, three or more identical complementarity determining regions in a single antibody (the actual number of complementarity determining regions/binding sites in a single antibody molecule is referred to as "valent"). For example, a single natural IgG antibody is monospecific and bivalent because it has two identical paratopes. Accordingly, a multispecific antibody comprises at least two (different) complementarity determining regions/binding sites. Therefore, the term "multispecific antibody" refers to an antibody that has more than one paratope and has the ability to bind to two or more different epitopes. The term "multispecific antibody" particularly includes the bispecific antibody as defined above, and generally also includes a protein, e.g., an antibody or a scaffold specifically binding to three or more different epitopes, i.e., an antibody having three or more paratopes/binding sites.

The term "valent" herein refers to the presence of a defined number of binding sites in an antibody/an antigen-binding molecule. Therefore, the terms "monovalent", "bivalent", "tetravalent" and "hexavalent" indicate the presence of one binding site, two binding sites, four binding sites and six binding sites in an antibody/antigen-binding molecule, respectively.

The "full-length antibody", "complete antibody" and "intact antibody" are used interchangeably herein and mean that they have a structure substantially similar to that of a natural antibody.

The "antigen-binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. Exemplarily, "antigen-binding fragment" or "antibody fragment" herein includes, but is not limited to, Fab, F(ab')2, Fab', Fab'-SH, Fd, Fv, scFv, diabody and a single domain antibody.

The term "chimeric antibody" herein refers to an antibody that has a variable sequence of an immunoglobulin from a source organism (such as a rat, a mouse, a rabbit or a llama) and a constant region of an immunoglobulin from a different organism (such as human). Methods for producing a chimeric antibody are known in the art. See, for example, Morrison, 1985, Science 229(4719): 1202-7; Oi et al., 1986, Bio Techniques 4:214-221; and Gillies et al., 1985 J Immunol Methods 125:191-202; which are incorporated herein by reference.

The term "humanized antibody" herein refers to a non-human antibody that has been genetically engineered, with amino acid sequences modified to improve the homology with the sequences of a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody usually retains or partially retains the expected properties of a donor antibody, including but not limited to, antigen specificity, affinity, reactivity, ability to enhance immune cell activity or ability to enhance immune response, etc.

The term "fully human antibody" herein refers to an antibody having variable regions in which both the FRs and CDRs are derived from human germline immunoglobulin sequences. Furthermore, if the antibody comprises a constant region, the constant region also is derived from human germline immunoglobulin sequences. The "fully human antibody" herein may include amino acid residues not encoded by human germline immunoglobulin sequences (for example, mutations introduced by random or site-specific mutagenesis in vitro or by somatic mutation in vivo). However, the "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains of a natural antibody generally have similar structures, and each domain comprises four conserved framework regions (FRs) and three hypervariable regions (HVRs). See, for example, Kindt et al., Kuby Immunology, 6th ed., W.H. Freeman and Co., p.91 (2007). A single VH or VL domain may be sufficient to confer antigen-binding specificity.

The terms "complementarity determining region" and "CDR" are used interchangeably herein and generally refer to hypervariable regions (HVRs) found in both light and heavy chain variable domains. The more conservative portion of a variable domain is called the framework region (FR). As understood in the art, the amino acid positions representing the hypervariable region of an antibody may vary according to the context and various definitions known in the art. Some positions within variable domains can be considered heterozygous hypervariable positions because these positions can be considered to be within the hypervariable regions under one set of criteria (such as IMGT or KABAT) but outside the hypervariable regions under a different set of criteria (such as KABAT or IMGT). One or more of these positions may also be found in extended hypervariable regions. The invention includes an antibody comprising modifications in these hybrid hypervariable positions. The heavy chain variable region CDR can be abbreviated as HCDR, and the light chain variable region CDR can be abbreviated as LCDR. The variable domains of a natural heavy chain and light chain each comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in order of FR1-CDR1-FR2-CDR2-FR3-CDR3-FR4 by the FR region, and together with the CDRs from other antibody chains, contribute to the formation of an antigen-binding site of an antibody (see Kabat et al., Sequences of Proteins of Immunological Interest, National Institute of Health, Bethesda, Md.1987; which is incorporated herein by reference).

For a further description of CDR, with reference to Kabat et al., J. Biol. Chem., 252:6609-6616 (1977); Kabat et al., United States Department of Health and Human Services, "Sequences of proteins of immunological interest" (1991); Chothia et al., J. Mol. Biol. 196:901-917 (1987); Al-Lazikani B. et al., J.Mol. Biol., 273: 927-948(1997); MacCallum et al., J. Mol.Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001). The "CDRs" herein can be marked and defined by well-known methods in the art, including but not limited to Kabat numbering system, Chothia numbering system or IMGT numbering system. The tool websites used include, but are not limited to, AbRSA website (http://cao.labshare.cn/AbRSA/cdrs.php), abYsis website (www.abysis.org/abysis/sequence_input/key_annotation/key_annotation.cgi) and IMGT website (http://www.imgt.org/3Dstructure-DB/cgi/DomainGapAlign.cgi#results). The CDRs herein include overlaps and subsets of amino acid residues defined in different ways.

The term "Kabat numbering system" herein generally refers to the immunoglobulin alignment and numbering system proposed by Elvin A. Kabat (see, for example, Kabat et al., Sequences of Proteins of Immunological Interest, 5th Ed. Public Health Service, National Institutes of Health, Bethesda, Md., 1991).

The term "Chothia numbering system" herein generally refers to the immunoglobulin numbering system proposed by Chothia *et al*., which is a classical rule for identifying the boundaries of CDR regions based on the location of structural loop regions (see, e.g., Chothia & Lesk (1987) J. Mol.Biol.196:901-917; Chothia et al. (1989) Nature 342:878-883).

The term "IMGT numbering system" herein generally refers to the numbering system based on The International ImMunoGeneTics Information System (IMGT) proposed by Lefranc *et al*., see Lefranc et al., Dev. Comparat. Immunol. 27:55-77, 2003.

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" is selected from a CH1 domain, a hinge region, a CH2 domain, a CH3 domain, or a variant or fragment thereof. The "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" is selected from Fc or a CH3 domain.

The term "light chain constant region" herein refers to the carboxyl terminus portion of an antibody light chain, which is not directly involved in the binding of an antibody to an antigen, and the light chain constant region is selected from a constant κ domain or a constant λ domain.

The term "Fc region" herein is used to define the C-terminal region of an antibody heavy chain that contains at least a portion of the constant region. The term includes a native sequence Fc region and a variant Fc region. Exemplarily, human IgG heavy chain Fc region can extend from Cys226 or Pro230 to the carboxyl terminus of the heavy chain. However, an antibody generated from a host cell may undergo post-translational cleavage whereby one or more, particularly one or two amino acids are cleaved off from the C-terminus of the heavy chain. Therefore, by expression of a specific nucleic acid molecule encoding a full-length heavy chain, the antibody generated from a host cell can include the full-length heavy chain, or a cleavage variant of the full-length heavy chain. This may be the case when the last two C-terminal amino acids of the heavy chain are glycine (G446) and lysine (K447, numbering according to the Kabat EU index). Therefore, the C-terminal lysine (Lys447), or the C-terminal glycine (Gly446) and lysine (Lys447) of the Fc region may or may not be present. Typically, an IgG Fc region comprises IgG CH2 and IgG CH3 domains, and optionally, on this basis, the IgG Fc region can also comprise a complete or partial hinge region, but does not comprise a CH1 domain. The "CH2 domain" of a human IgG Fc region generally extends from an amino acid residue at about position 231 to an amino acid residue at about position 340. In one embodiment, a carbohydrate chain is attached to the CH2 domain. The CH2 domain herein can be a native sequence CH2 domain or a variant CH2 domain. The "CH3 domain" comprises the stretch of residues at the C-terminus of the CH2 domain in the Fc region (i.e., from an amino acid residue at about position 341 to an amino acid residue at about position 447 of IgG). The CH3 region herein can be a native sequence CH3 domain or a variant CH3 domain (e.g., a CH3 domain having a "knob" introduced in one chain thereof and a "hole" correspondingly introduced in the other chain thereof; see US Patent No. 5,821,333, which is expressly incorporated herein by reference). As described herein, such variant CH3 domains can be used to facilitate heterodimerization of two non-identical antibody heavy chains.

Unless otherwise specified herein, the numbering of amino acid residues in an Fc region or a constant region is according to the EU numbering system, also known as the EU index, as described in Sequences of Proteins of Immunological Interest, 5th Ed. Public Health Service, National Institutes of Health, Bethesda, MD, 1991.

The term "Fc variant" herein refers to changes in the structure or function of Fc caused by one or more amino acid substitution, insertion or deletion mutations at appropriate positions on the Fc. The "interaction between Fc variants" refers to the space-filling effect, electrostatic steering, hydrogen bonding effect, hydrophobic effect, etc. formed between Fc variants that have been subjected to a mutation design. The interaction between Fc variants contributes to the formation of a stable heterodimeric protein. A preferred mutation design is a mutation design in a "Knob-into-Hole" form.

The mutation design technique of Fc variants has been widely used in the field to prepare bispecific antibodies or heterodimeric Fc fusion protein forms. Representative ones include the "Knob-into-Hole" form proposed by Cater et al. (Protein Engineering vol.9 no.7 pp. 617-621, 1996); an Fc-containing heterodimer form formed by using Electrostatic Steering by the technician in Amgen (US 20100286374 A1); The heterodimer form (SEEDbodies) formed by IgG/Ig chain exchange proposed by Jonathan H. Davis et al. (Protein Engineering, Design & Selection pp. 1-8, 2010); Bispecific molecules formed by Genmab's DuoBody (Science, 2007.317(5844)) platform technology; heterodimeric protein forms formed by combining structural calculation and Fc amino acid mutations and combining different modes of action by Xencor's technical staff (mAbs 3: 6, 546-557; November/December 2011); a heterodimeric protein form obtained by the charge network-based Fc modification method of Alphamab Oncology (CN 201110459100.7); and other genetic engineering methods for forming heterodimeric functional proteins based on Fc amino acid changes or functional modification means. The Knob/Hole structure on the Fc variant fragments of the present invention means that the two Fc fragments are mutated respectively, and can be combined in a "Knob-into-Hole" form after the mutations. Preferably, the Fc regions are subjected to site mutation modification using the "Knob-into-hole" model of Cater et al., so that the obtained first Fc variant and second Fc variant can be combined together in a "Knob-into-hole" form to form a heterodimer. The selection of particular immunoglobulin Fc regions from particular immunoglobulin classes and subclasses is within the range of those skilled in the art. The Fc regions of human antibodies IgG1, IgG2, IgG3 and IgG4 are preferred, and the Fc region of human antibody IgG1 is more preferred. Either of the first Fc variant or the second Fc variant is randomly selected for knob mutation and the other for hole mutation.

The term "conservative amino acid" herein generally refers to amino acids that belong to the same class or have similar characteristics (such as charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method: To determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position. The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needleman and Wunsch algorithm ((1970) J. Mol. Biol. 48: 444-453) in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70 or 80 and a length weight of 1, 2, 3, 4, 5 or 6. A particularly preferred parameter set (and one parameter set that should be used unless otherwise stated) is the Blossum62 scoring matrix with a gap penalty of 12, a gap extension penalty of 4, and a gap frameshift penalty of 5. The percent identity between two amino acid sequences or nucleotide sequences can also be determined using the PAM120 weighted remainder table, with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid and protein sequences of the present invention can further be used as "query sequences" to perform searches against public databases, e.g., to identify other family member sequences or related sequences. For example, the NBLAST and XBLAST programs (version 2.0) of Altschul et al., (1990) J. Mol. Biol. 215: 403-10 can be used to perform such searches. BLAST nucleotide searches can be performed with the NBLAST program, with score = 100 and word length = 12, to obtain the nucleotide sequences homologous to the nucleic acid molecules of the present invention. BLAST protein searches can be performed with the XBLAST program, with score = 50 and word length = 3, to obtain the amino acid sequences homologous to the protein molecules of the present invention. To obtain gapped alignment results for comparison purposes, gapped BLAST can be used as described in Altschul et al., (1997) Nucleic Acids Res. 25: 3389-3402. When BLAST and gapped BLAST programs are used, the default parameters of the corresponding programs (e.g., XBLAST and NBLAST) can be used. See www.ncbi.nlm.nih.gov.

The term "nucleic acid" herein includes any compound and/or substance comprising a polymer of nucleotides. Each nucleotide consists of a base, specifically a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the invention *in vitro* and/or *in vivo*, for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody in vivo (see, for example, Stadler et al., Nature Medicine 2017, Published online June 12, 2017, doi: 10.1038/nm.4356 or EP 2101823B1).

The "isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of the nucleic acid to which they are operably linked. Such vectors are referred to herein as "expression vectors".

The term "host cell" herein refers to a cell into which a foreign nucleic acid is introduced, including the progenies of such a cell. The host cell includes "transformant" and "transformed cell" which include the primary transformed cell and progeny derived therefrom, regardless of the number of passages. The progeny may not be identical to the parental cell in nucleic acid content, and may contain a mutation. The mutant progeny with the same function or biological activity as those screened or selected in the initially transformed cells are included herein.

As used herein, the term "pharmaceutical composition" refers to a preparation that is present in a form which allows the active ingredients contained therein to be biologically effective and does not contain additional ingredients that would be unacceptably toxic to the subject to which the pharmaceutical composition is administered.

The term "pharmaceutically acceptable carrier" herein includes any and all solvents, dispersion media, coating materials, surfactants, antioxidants, preservatives (e.g., antibacterial agents and antifungal agents), isotonic agents, absorption delaying agents, salts, preservatives, drug stabilizers, binders, excipients, disintegrants, lubricants, sweeteners, flavoring agents, dyes, etc., and a combination thereof, which are known to those skilled in the art (see, for example, Remington's Pharmaceutical Sciences, 18th ed., Mack Printing Company, 1990, pp. 1289-1329). Except in cases of incompatibility with the active ingredient, any conventional carrier is contemplated for use in a therapeutic or pharmaceutical composition.

The term "treatment" herein refers to surgical or therapeutic treatment, the purpose of which is to prevent and slow down (reduce) an undesired physiological change or pathology in a subject being treated, such as cancers and tumors. Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

The term "subject" refers to an organism receiving treatment for a particular disease or condition as described herein. Exemplarily, a "subject" includes a mammal, such as a human, a primate (e.g., monkey) or a non-primate mammal, receiving treatment for a disease or a condition.

The term "effective amount" herein refers to an amount of a therapeutic agent effective to prevent or relieve a disease or a disorder or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When the active ingredient is administered to an individual alone, a therapeutically effective dose refers to the ingredient alone. When a certain combination is applied, a therapeutically effective dose refers to the combined dosage of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Detailed Description of Embodiments

The present application provides an antibody or an antigen-binding fragment thereof that specifically binds to BCMA, a multispecific antigen-binding molecule, a chimeric antigen receptor, an immune effector cell, a nucleic acid fragment, a vector, a host cell, a pharmaceutical composition, a kit, a preparation method, and the use of the antibody or the antigen-binding fragment thereof in the treatment of diseases and detection of BCMA.

In a first aspect, the present application provides an antibody or an antigen-binding fragment thereof that specifically binds to BCMA, wherein the antibody or the antigen-binding fragment thereof comprises CDR1, CDR2 and CDR3, the CDR1 comprises HCDR1 of VHH domain shown in any sequence of SEQ ID NO: 7-21, 24-31 and 92-101, the CDR2 comprises HCDR2 of VHH domain shown in any sequence of SEQ ID NO: 7-21, 24-31 and 92-101,and the CDR3 comprises HCDR3 of VHH domain shown in any sequence of SEQ ID NO: 7-21, 24-31 and 92-101.

In some specific embodiments, the HCDR1, HCDR2 and HCDR3 are determined according to the Kabat, IMGT or Chothia numbering system.

In an optional embodiment, the HCDR1, HCDR2 and HCDR3 are selected from the amino acid sequences as represented by SEQ ID NO: 34-91 and 102-157.

In an optional embodiment, the HCDR1 comprises the amino acid sequence as represented by SEQ ID NOs: 34, 37, 40, 42, 45, 48, 50, 52, 54, 56, 58, 64, 67, 71, 74, 77, 81, 84, 86, 87, 104, 107, 113, 116, 119, 121, 124, 127, 131, 134, 138 or 141.

In an optional embodiment, the HCDR2 comprises the amino acid sequence as represented by SEQ ID NOs: 35, 38, 41, 43, 46, 49, 59, 61, 63, 65, 68, 70, 72, 75, 78, 79, 82, 85, 88, 89, 90, 91, 102, 105, 108, 109, 111, 114, 117, 120, 122, 125, 128, 129, 132, 135, 136, 139, 142, 143, 148, 150, 152, 153, 155 or 157.

In an optional embodiment, the HCDR3 comprises the amino acid sequence as represented by SEQ ID NO: 36, 39, 44, 47, 51, 53, 55, 57, 60, 62, 66, 69, 73, 76, 80, 83, 103, 106, 110, 112, 115, 118, 123, 126, 130, 133, 137, 140, 144, 145, 146, 147, 149, 151, 154 or 156.

In a preferred embodiment, according to the Kabat, IMGT or Chothia numbering system, the HCDR1, HCDR2 and HCDR3 comprise the amino acid sequences as represented by SEQ ID NOs: 34, 35 and 36; SEQ ID NOs: 37, 38 and 39; or SEQ ID NOs: 40, 41 and 36, respectively.

In a preferred embodiment, according to the Kabat, IMGT or Chothia numbering system, the HCDR1, HCDR2 and HCDR3 comprise the amino acid sequences as represented by SEQ ID NOs: 42, 43 and 44; SEQ ID NOs: 45, 46 and 47; or SEQ ID NOs: 48, 49 and 44, respectively.

In a preferred embodiment, according to the Kabat, IMGT or Chothia numbering system, the HCDR1, HCDR2 and HCDR3 comprise the amino acid sequences as represented by SEQ ID NOs: 50, 35 and 51; SEQ ID NOs: 52, 38 and 53; or SEQ ID NOs: 54, 41 and 51, respectively.

In a preferred embodiment, according to the Kabat, IMGT or Chothia numbering system, the HCDR1, HCDR2 and HCDR3 comprise the amino acid sequences as represented by SEQ ID NOs: 34, 35 and 55; SEQ ID NOs: 56, 38 and 57; or SEQ ID NOs: 58, 41 and 55, respectively.

In a preferred embodiment, according to the Kabat, IMGT or Chothia numbering system, the HCDR1, HCDR2 and HCDR3 comprise the amino acid sequences as represented by SEQ ID NOs: 34, 59 and 60; SEQ ID NOs: 56, 61 and 62; or SEQ ID NOs: 58, 63 and 60, respectively.

In a preferred embodiment, according to the Kabat, IMGT or Chothia numbering system, the HCDR1, HCDR2 and HCDR3 comprise the amino acid sequences as represented by SEQ ID NOs: 64, 65 and 66; SEQ ID NOs: 67, 68 and 69; or SEQ ID NOs: 58, 70 and 66, respectively.

In a preferred embodiment, according to the Kabat, IMGT or Chothia numbering system, the HCDR1, HCDR2 and HCDR3 comprise the amino acid sequences as represented by SEQ ID NOs: 71, 72 and 73; SEQ ID NOs: 74, 75 and 76; or SEQ ID NOs: 77, 78 and 73, respectively.

In a preferred embodiment, according to the Kabat, IMGT or Chothia numbering system, the HCDR1, HCDR2 and HCDR3 comprise the amino acid sequences as represented by SEQ ID NOs: 34, 79 and 80; SEQ ID NOs: 81, 82 and 83; or SEQ ID NOs: 84, 85 and 80, respectively.

In a preferred embodiment, according to the Kabat numbering system, the HCDR1, HCDR2 and HCDR3 comprise the amino acid sequences as represented by SEQ ID NOs: 86, 35 and 36 respectively.

In a preferred embodiment, according to the Kabat numbering system, the HCDR1, HCDR2 and HCDR3 comprise the amino acid sequences as represented by SEQ ID NOs: 87, 88 and 44 respectively.

In a preferred embodiment, according to the Kabat numbering system, the HCDR1, HCDR2 and HCDR3 comprise the amino acid sequences as represented by SEQ ID NOs: 42, 88 and 44 respectively.

In a preferred embodiment, according to the Kabat numbering system, the HCDR1, HCDR2 and HCDR3 comprise the amino acid sequences as represented by SEQ ID NOs: 42, 89 and 44 respectively.

In a preferred embodiment, according to the Kabat numbering system, the HCDR1, HCDR2 and HCDR3 comprise the amino acid sequences as represented by SEQ ID NOs: 42, 90 and 44 respectively.

In a preferred embodiment, according to the Kabat numbering system, the HCDR1, HCDR2 and HCDR3 comprise the amino acid sequences as represented by SEQ ID NOs: 42, 91 and 44 respectively.

In a preferred embodiment, according to the Kabat, IMGT or Chothia numbering system, the HCDR1, HCDR2 and HCDR3 comprise the amino acid sequences as represented by SEQ ID NOs: 71, 102 and 103; SEQ ID NOs: 104, 105 and 106; or SEQ ID NOs: 107, 108 and 103, respectively.

In a preferred embodiment, according to the Kabat, IMGT or Chothia numbering system, the HCDR1, HCDR2 and HCDR3 comprise the amino acid sequences as represented by SEQ ID NOs: 71, 109 and 110; SEQ ID NOs: 104, 111 and 112; or SEQ ID NOs: 107, 108 and 110, respectively.

In a preferred embodiment, according to the Kabat, IMGT or Chothia numbering system, the HCDR1, HCDR2 and HCDR3 comprise the amino acid sequences as represented by SEQ ID NOs: 113, 114 and 115; SEQ ID NOs: 116, 117 and 118; or SEQ ID NOs: 119, 120 and 115, respectively.

In a preferred embodiment, according to the Kabat, IMGT or Chothia numbering system, the HCDR1, HCDR2 and HCDR3 comprise the amino acid sequences as represented by SEQ ID NOs: 121, 122 and 123; SEQ ID NOs: 124, 125 and 126; or SEQ ID NOs: 127, 128 and 123, respectively.

In a preferred embodiment, according to the Kabat, IMGT or Chothia numbering system, the HCDR1, HCDR2 and HCDR3 comprise the amino acid sequences as represented by SEQ ID NOs: 113, 129 and 130; SEQ ID NOs: 131, 132 and 133; or SEQ ID NOs: 134, 135 and 130, respectively.

In a preferred embodiment, according to the Kabat, IMGT or Chothia numbering system, the HCDR1, HCDR2 and HCDR3 comprise the amino acid sequences as represented by SEQ ID NOs: 113, 136 and 137; SEQ ID NOs: 138, 139 and 140; or SEQ ID NOs: 141, 142 and 137, respectively.

In a preferred embodiment, according to the Kabat, IMGT or Chothia numbering system, the HCDR1, HCDR2 and HCDR3 comprise the amino acid sequences as represented by SEQ ID NOs: 34, 143 and 144; SEQ ID NOs: 37, 38 and 145; or SEQ ID NOs: 40, 41 and 144, respectively.

In a preferred embodiment, according to the Kabat, IMGT or Chothia numbering system, the HCDR1, HCDR2 and HCDR3 comprise the amino acid sequences as represented by SEQ ID NOs: 34, 143 and 146; SEQ ID NOs: 56, 38 and 147; or SEQ ID NOs: 58, 41 and 146, respectively.

In a preferred embodiment, according to the Kabat, IMGT or Chothia numbering system, the HCDR1, HCDR2 and HCDR3 comprise the amino acid sequences as represented by SEQ ID NOs: 34, 148 and 149; SEQ ID NOs: 37, 150 and 151; or SEQ ID NOs: 40, 152 and 149, respectively.

In a preferred embodiment, according to the Kabat, IMGT or Chothia numbering system, the HCDR1, HCDR2 and HCDR3 comprise the amino acid sequences as represented by SEQ ID NOs: 71, 153 and 154; SEQ ID NOs: 104, 155 and 156; or SEQ ID NOs: 107, 157 and 154, respectively.

In some specific embodiments, the CDR1, CDR2 and/or CDR3 comprise(s) an amino acid sequence with 1, 2 or 3 mutations on the aforementioned HCDR1, HCDR2 and/or HCDR3; the mutations are selected from insertion, deletion and/or substitution, and the substitution is preferably a conservative amino acid substitution.

In some specific embodiments, the CDR1, CDR2 and/or CDR3 comprise(s) an amino acid sequence having at least 80%, 85%, 90%, 91%, 92%, 93%, 94%, 95%, 96%, 97%, 98%, 99% or 100% identity to the aforementioned HCDR1, HCDR2 and/or HCDR3.

In some specific embodiments, the antibody or the antigen-binding fragment thereof comprises a single domain antibody, and the single domain antibody comprises the aforementioned CDR1, CDR2 and CDR3.

In some specific embodiments, the single domain antibody comprises an amino acid sequence selected from any one of SEQ ID NO: 7-21, 24-31, and 92-101.

In an optional embodiment, the single domain antibody comprises an amino acid sequence with at most 20, 19, 18, 17, 16, 15, 14, 13, 12, 11, 10, 9, 8, 7, 6, 5, 4, 3, 2 or 1 mutation relative to the sequence as shown in any one of SEQ ID NOs: 7-21, 24-31 and 92-101, the mutation is selected from insertion, deletion and/or substitution, and the substitution is preferably a conservative amino acid substitution.

In an optional embodiment, the single domain antibody comprises an amino acid sequence having at least 80%, 85%, 90%, 91%, 92%, 93%, 94%, 95%, 96%, 97%, 98%, 99% or 100% identity to the sequence as shown in any one of SEQ ID NOs: 7-21, 24-31 and 92-101.

In some specific embodiments, the antibody comprises an FR region in a VHH domain as shown in any one of SEQ ID NOs: 7-21, 24-31 and 92-101.

In an optional embodiment, the antibody comprises an amino acid sequence with at most 15, 14, 13, 12, 11, 10, 9, 8, 7, 6, 5, 4, 3, 2 or 1 mutation relative to the FR region in the VHH domain as shown in any one of SEQ ID NOs: 7-21, 24-31 and 92-101, the mutation is selected from insertion, deletion and/or substitution, and the substitution is preferably a conservative amino acid substitution;

In an optional embodiment, the antibody comprises an amino acid sequence having at least 80%, 85%, 90%, 91%, 92%, 93%, 94%, 95%, 96%, 97%, 98%, 99% or 100% identity to the FR region in the VHH domain as shown in any one of SEQ ID NOs: 7-21, 24-31 and 92-101.

In some specific embodiments, the antibody or the antigen-binding fragment thereof is selected from: (1) a chimeric antibody or a fragment thereof; (2) a humanized antibody or a fragment thereof; or (3) a fully human antibody or a fragment thereof.

In some specific embodiments, the antibody or the antigen-binding fragment thereof comprises or does not comprise an antibody heavy chain constant region; optionally, the antibody heavy chain constant region is selected from humans, llamas, mice, rats, rabbits and goats; optionally, the antibody heavy chain constant region is selected from IgG, IgM, IgA, IgE and IgD; optionally, the IgG is selected from IgG1, IgG2, IgG3 and IgG4; optionally, the heavy chain constant region is selected from an Fc region, a CH3 region and a complete heavy chain constant region, preferably, the heavy chain constant region is a human Fc region; and preferably, the antibody or the antigen-binding fragment thereof is a heavy chain antibody.

In some specific embodiments, the antibody or the antigen-binding fragment thereof is further coupled with a therapeutic agent or a tracer; preferably, the therapeutic agent is selected from a radioisotope, a chemotherapeutic drug and an immunomodulator, and the tracer is selected from a radiological contrast agent, a paramagnetic ion, a metal, a fluorescent tag, a chemiluminescence tag, an ultrasonic contrast agent and a photosensitizer.

In some specific embodiments, the antibody or the antigen-binding fragment thereof is also linked to other functional molecules, and preferably, the other functional molecules are selected from one or more of the following: a signal peptide, a protein tag, a cytokine, an angiogenesis inhibitor and an immune checkpoint inhibitor.

In a second aspect, the present application further provides a multispecific antigen-binding molecule, wherein the multispecific antigen-binding molecule comprises the aforementioned antibody or the antigen-binding fragment thereof, and an antigen-binding molecule binding to an antigen other than BCMA or binding to a different BCMA epitope than that of the aforementioned antibody or the antigen-binding fragment thereof; optionally, the antigen other than BCMA is selected from: CD3 (preferably CD3ε), CD16, CD137, CD258, PD-1, PD-L1, 4-1BB, CD40, CD64, EGFR, VEGF, HER2, HER1, HER3, IGF-1R, phosphatidylserine (PS), C-Met, HSA, GPRC5D, MSLN, blood-brain barrier receptor, GPC3, PSMA, CD33, GD2, ROR1, ROR2, FRα and Gucy2C.

In a preferred embodiment, the antigen-binding molecule is an antibody or an antigen-binding fragment thereof.

In a preferred embodiment, the multispecific antigen-binding molecule can be bispecific, trispecific or tetraspecific.

In a preferred embodiment, the multispecific antigen-binding molecule can be bivalent, trivalent, tetravalent, pentavalent or hexavalent.

In a third aspect, the present application further provides an isolated nucleic acid fragment, wherein the nucleic acid fragment encodes the aforementioned antibody or the antigen-binding fragment thereof or the multispecific antigen-binding molecule.

In a fourth aspect, the present application also provides a vector, which comprises the aforementioned nucleic acid fragment.

In a fifth aspect, the present application further provides a host cell, which comprises the aforementioned vector; and preferably, the cell is a prokaryotic cell or a eukaryotic cell, such as a bacterial (for example, *Escherichia coli*) cell, a fungal (for example, yeast) cell, an insect cell or a mammalian cell (for example, a CHO cell line or a 293T cell line).

In a sixth aspect, the present application further provides a method for preparing the antibody or the antigen-binding fragment thereof or the aforementioned multispecific antigen-binding molecule, wherein the method comprises culturing the aforementioned cell, and isolating the antibody, antigen-binding fragment or multispecific antigen-binding molecule expressed by the cell.

In the seventh aspect, the present application further provides a pharmaceutical composition, wherein the pharmaceutical composition comprises the aforementioned antibody or the antigen-binding fragment thereof, multispecific antigen-binding molecule, nucleic acid fragment or vector, or a product prepared according to the aforementioned method; optionally, the pharmaceutical composition also comprises a pharmaceutically acceptable carrier, a diluent or an auxiliary agent; and optionally, the pharmaceutical composition further comprises an additional anti-tumor agent.

In some embodiments, the pharmaceutically acceptable carrier is a carrier that does not weaken the viability and function of immune cells and does not affect the specific binding of antibodies or antigen-binding fragments thereof to antigens, and includes but not limited to cell culture media, buffers, physiological saline, balanced salt solution, *etc.* Examples of buffers include isotonic phosphate, acetate, citrate, borate, carbonate, *etc.* In specific embodiments, the pharmaceutically acceptable carrier is phosphate buffered saline containing 1% serum.

The anti-tumor agents are, for example, various chemotherapeutic drugs, such as alkylating agent chemotherapeutic drugs, including but not limited to cyclophosphamide, ifosfamide, melphalan, carmustine, lomustine, nimustine, fotemustine, *etc.;* Anti-metabolite chemotherapeutic drugs, including but not limited to methotrexate, fluorouracil, tegafur, carmofur, deoxyfloxuridine, capecitabine, gemcitabine, raltitrexed, *etc.;* Anti-cancer antibiotics, including but not limited to actinomycin D, mitomycin, bleomycin, daunorubicin, doxorubicin, epirubicin, pirarubicin, *etc.;* Plant-based chemotherapeutic drugs, including but not limited to vincristine, vindesine, vinorelbine, irinotecan, topotecan, paclitaxel, docetaxel, *etc.;* Miscellaneous others, including but not limited to dacarbazine, cisplatin, carboplatin, oxaliplatin, nedaplatin, *etc.*

In an eighth aspect, the present application further provides a method for treating a tumor or a cancer, wherein the method comprises administering to a subject an effective amount of the aforementioned antibody or antigen-binding fragment thereof, the multispecific antigen-binding molecule, the nucleic acid fragment, the vector, a product prepared according to the aforementioned method or the aforementioned pharmaceutical composition; optionally, the tumor or cancer is a BCMA-expressing tumor or cancer, preferably a B-cell lymphoma, and more preferably multiple myeloma (MM).

In a ninth aspect, the present application further provides the use of the aforementioned antibody or the antigen-binding fragment thereof, the multispecific antigen-binding molecule, the nucleic acid fragment, the vector, a product prepared according to the aforementioned method or the aforementioned pharmaceutical composition in the preparation of a drug for treating a tumor or a cancer; optionally, the tumor or cancer is a BCMA-expressing tumor or cancer, preferably a B-cell lymphoma, and more preferably multiple myeloma (MM).

In a tenth aspect, the present application further provides the use of the aforementioned antibody or the antigen-binding fragment thereof, the multispecific antigen-binding molecule, the nucleic acid fragment, the vector, a product prepared according to the aforementioned method or the aforementioned pharmaceutical composition for treating a tumor or a cancer; optionally, the tumor or cancer is a BCMA-expressing tumor or cancer, preferably a B-cell lymphoma, and more preferably multiple myeloma (MM).

In an eleventh aspect, the present application further provides a kit, wherein the kit comprises the aforementioned antibody or the antigen-binding fragment thereof, the multispecific antigen-binding molecule, the nucleic acid fragment, the vector, a product prepared according to the aforementioned method or the aforementioned pharmaceutical composition.

In a twelfth aspect, the present application further provides a method for detecting BCMA expression in a biological sample, wherein the method comprises contacting the biological sample with the aforementioned antibody or the antigen-binding fragment thereof under conditions that allow the formation of a complex from the antibody or the antigen-binding fragment thereof and BCMA; and preferably, the method further comprises detecting the formation of the complex, thereby indicating the presence or expression level of BCMA in the sample.

In a thirteenth aspect, the present application further provides the use of the aforementioned antibody or the antigen-binding fragment thereof in preparing a BCMA detection reagent.

### Examples

The present application will be further described below in conjunction with specific examples, and the advantages and characteristics of the present application will become clearer along with the description. If no specific conditions are indicated in the examples, conventional conditions or the conditions suggested by the manufacturer shall be followed. Any reagents or instruments used, unless the manufactures stated, are conventional products that are commercially available.

The examples herein are only exemplary and do not limit the scope of the present application in any way. Those skilled in the art should understand that the details and forms of the technical solutions of the present application can be modified or replaced without departing from the spirit and scope of the present application, but these modifications and replacements all fall within the scope of protection of the present application.

### Example 1 Preparation of control antibodies, identification of endogenous cells and preparation of overexpression cell lines

### (A) Preparation of control antibodies

REGN5459 and HPN217 are both antibodies that recognize human BCMA protein. The sequence of REGN5459 comes from US Patent Publication No. US 2020/0024356A1, and the HPN217 sequence comes from US Patent Publication No. US 20190112381A1. The heavy chain variable region (VH) of REGN5459 was recombined into an expression vector containing a signal peptide and human antibody IgG1 heavy chain constant region, and the light chain variable region (VL) sequence was recombined into an expression vector containing a signal peptide and human antibody IgG1 light chain, thus a recombinant plasmid was obtained and the synthesized antibody was named REGN5459-hIgG1. The VHH sequence of HPN217 was recombined into an expression vector containing a signal peptide and human antibody IgG1 Fc to obtain a recombinant plasmid. The synthesized antibody was named HPN217-hHcAb. The negative control antibody hIgG1 was the antibody anti-hel-hIgG1 (purchased from Biointron, catalog number: B11790 1, hereinafter referred to as hIgG 1) against Hen Egg Lysozyme chicken egg lysozyme.

**Table 1 List of sequences of control antibodies**

| **Design ation of sequen ce** | **Sequen ce numbe r** | **Amino acid sequence** |
|---|---|---|
| REGN5 459 VH | SEQ ID NO: 1 | EVQLVESGGGLVQPGGSLRLSCAASGFTFSNFWMTWVRQA PGKGLEWVANMNQDGSEKYYVDSVKGRFTISRDNAKSSL YLQMNSLRAEDTAVYYCARDREYCISTSCYDDFDYWGQG TLVTVSS |
| REGN5 459 VL | SEQ ID NO: 2 | DIQMTQSPSSLSASVGDRVTITCRASQSISSYLNWYQQKPG KAPKLLIYAASSLQSGVPSRFSGSGSGTDFTLTISSLQPEDFA TYYCQQSYSTPPITFGQGTRLEIK |
| HPN21 7 VHH | SEQ ID NO: 3 | EVQLVESGGGLVQPGRSLTLSCAASTNIFSISPYGWYRQAPG KQRELVAAIHGTSTLYADSVKGRFTISRDNAKNSIYLQMNS LRPEDTALYYCNKVPWGDYHPGNVYWGQGTQVTVSS |
| IgG1 Fc | SEQ ID NO: 4 | EPKSSDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSREEMTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| IgG1 heavy chain constant region | SEQ ID NO: 5 | ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPELLGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALPAPIEKTISKAKGQPREPQVYTLPPSREEMTKN QVSLTCLVKGFYPSDIAVEWESNGQPENNYKTTPPVLDSDG SFFLYSKLTVDKSRWQQGNVFSCSVMHEALHNHYTQKSLS LSPGK |
| IgG1 light chain constant region | SEQ ID NO: 6 | |

| | | |
|---|---|---|
| Notes: The IgG1 Fc contains the C220S mutation. | | |

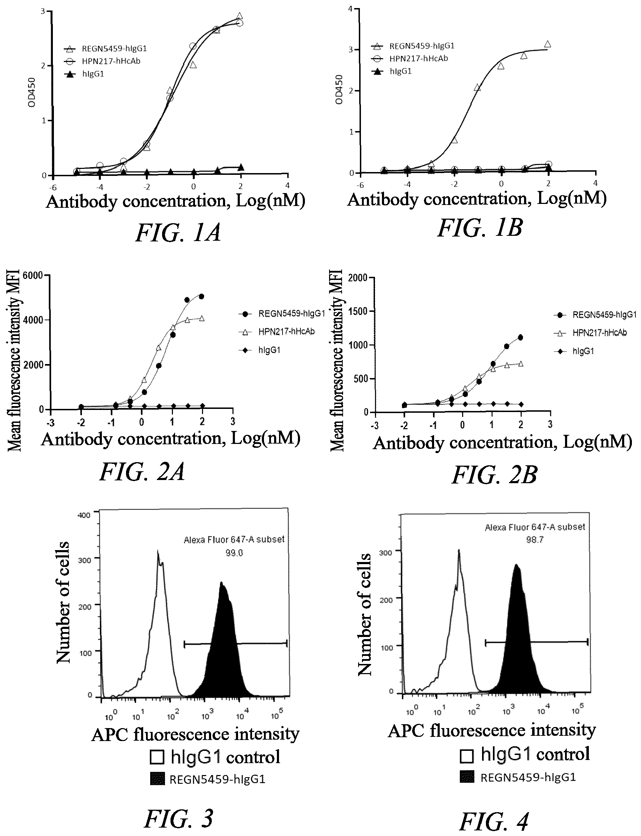

The binding activity of the control antibody to human BCMA-His protein (purchased from Aero, catalog number: BCA-H522y) and monkey BCMA-His protein (purchased from Aero, catalog number: BCA-C52H7) was detected by ELISA. The specific method was as follows: The antigen protein was diluted with PBS to a final concentration of 1 µg/mL, and then added to a 96-well ELISA plate at 50 µl per well. The plate was sealed with a plastic film and incubated overnight at 4°C, the plate was washed twice with PBS the next day, and then a blocking solution [PBS + 2% (w/v) BSA] was added for blocking at room temperature for 2 h. The blocking solution was poured off and 50 µl of 100 nM serially diluted control antibody or negative control antibody was added to each well. After incubation at 37°C for 2 h, the plate was washed 3 times with PBS. HRP (horseradish peroxidase)-labeled secondary antibody (purchased from Merck, catalog number: AP113P) was added, and incubated at 37°C for 1 h, and the plate was washed 5 times with PBS. TMB substrate was added at 50 µl/well, and incubated at room temperature for 10 min, then a stop solution (1.0 M HCl) was added at 50 µl/well. The OD450 nm values were read by an ELISA microplate reader (Multimode Plate Reader, EnSight, purchased from Perkin Elmer). The results are shown in Tables 2 and 3 and Figs. 1A and 1B. REGN5459-hIgG1 antibody had good binding activity to both human BCMA protein and monkey BCMA protein. HPN217-hHcAb had good binding activity to human BCMA protein, but did not bind to monkey BCMA protein.

**Table 2 ELISA detection of binding activity of control antibody with human BCMA-his protein**

| **Designation of antibody** | **Antibody concentration (nM)** | | | | | | | |
|---|---|---|---|---|---|---|---|---|
| | **100** | **10** | **1** | **0.1** | **0.01** | **0.001** | **0.0001** | **Blank control** |
| REGN5459-hIgG1 | 2.89 | 2.63 | 2.00 | 1.54 | 0.50 | 0.14 | 0.07 | 0.06 |
| HPN217-hHcAb | 2.75 | 2.64 | 2.34 | 1.39 | 0.56 | 0.24 | 0.17 | 0.07 |
| hIgG1 | 0.12 | 0.07 | 0.05 | 0.05 | 0.05 | 0.05 | 0.05 | 0.06 |

**Table 3 ELISA detection of binding activity of control antibody with monkey BCMA-his protein**

| **Designation of antibody** | **Antibody concentration (nM)** | | | | | | | |
|---|---|---|---|---|---|---|---|---|
| | **100** | **10** | **1** | **0.1** | **0.01** | **0.001** | **0.0001** | **Blank control** |
| REGN5459-hIgG1 | 3.10 | 2.82 | 2.57 | 2.06 | 0.78 | 0.21 | 0.09 | 0.06 |
| HPN217-hHcAb | 0.16 | 0.06 | 0.05 | 0.05 | 0.05 | 0.05 | 0.05 | 0.05 |
| hIgG1 | 0.10 | 0.06 | 0.05 | 0.05 | 0.04 | 0.05 | 0.05 | 0.05 |

### (B) Identification of cell lines expressing BCMA protein endogenously

H929 cells and U266 cells were scale-up cultured in T-75 cell culture flasks to the logarithmic growth phase, and the cells were pipetted into a single cell suspension. After the cells were counted, the cells were centrifuged and the cell pellet was resuspended in FACS buffer (PBS + 2% fetal calf serum) to 2 × 10⁶ cells per ml, and the suspension was added to the 96-well FACS reaction plate at 50 µl per well, REGN5459-hIgG1 and HPN217-hHcAb antibodies were used as primary antibodies, and APC-labeled goat anti-human IgG (H+L) secondary antibodies (purchased from Jackson, catalog number: 109-605-088) were detected and analyzed by FACS (FACS CantoTM, purchased from BD Corporation). The results are shown in Table 4 and Figs. 2A and 2B, and indicate that both H929 cells and U266 cells can bind to REGN5459-hIgG1 and HPN217-hHcAb antibodies.

**Table 4 FACS detection results of endogenous cell lines H929 and U266**

| **Designation of antibody** | **H929** | | **U266** | |
|---|---|---|---|---|
| | **Maximum mean fluorescence intensity** | **EC50 (nM)** | **Maximum mean fluorescence intensity** | **EC50 (nM)** |
| REGN5459-hIgG1 | 4993 | 6.78 | 1097 | 9.12 |
| HPN217-hHcAb | 4015 | 2.24 | 708 | 1.83 |
| hIgG1 | 110 | Negative | 103 | Negative |

### (C) Preparation of Flp-inCHO recombinant cell lines of human BCMA protein and monkey BCMA protein

The nucleotide sequences encoding the amino acid sequences of human BCMA and monkey BCMA were cloned into pcDNA5/FRT vector (purchased from Clontech) respectively. Human BCMA NCBI Gene ID: 608, Monkey BCMA NCBI Gene ID: 712212. The Flp-inCHO cell lines (purchased from the cell bank of the Type Culture Collection Committee of Chinese Academy of Sciences) were transfected (Lipofectamine^{®} 3000 Transfection Kit, purchased from Invitrogen, catalog number: L3000-015) respectively, and then selectively cultured in F12K Medium (Gibco, catalog number: 21127030) containing 600 µg/ml hygromycin (ThermoFisher, catalog number: 10687010) and 10% (v/v) fetal bovine serum (ExCell Bio, catalog number: FND500) for 2 weeks. REGN5459-hIgG1 and goat anti-human IgG(H+L) antibody (Jackson, catalog number: 109605088) were used for detection on FACS CantoII (purchased from BD Biosciences), and the cells with high expression and single peak shape were proliferated, and the proliferated cells were retested by flow cytometry. The positive cell pool with better growth, higher fluorescence intensity and better homogeneity was selected for further scale-up culture and then cryopreserved in liquid nitrogen. Table 5 shows that a Flp-inCHO high-expression cell population with human BCMA-positive expression and a Flp-inCHO high-expression cell population with monkey BCMA-positive expression have been prepared respectively. In Figs. 3 and 4, the abscissa was the fluorescence intensity of cells, and the ordinate was the number of cells.

**Table 5 FACS detection results of Flp-inCHO recombinant cell line expressing human BCMA protein**

| **No.** | **Stably transfected positive cell population** | **Cell mean fluorescence density** | |
|---|---|---|---|
| | | **IgG subtype control** | **BCMA antibody** |
| 1 | Flp-in CHO-human BCMA | 54.6 | 4313 |
| 2 | Flp-in CHO-monkey BCMA | 49 | 2984 |

### Example 2 Preparation of alpaca nanobody against BCMA

### (A) Animal immunization and serum titer determination

The immunized animal was an alpaca (Alpaca, No. NB254). Before immunization, 10 mL of blood was taken as a negative control. 0.5 mg of human BCMA(Met1- Ala54)-hFc protein (purchased from Aero, catalog number: BC7-H5254) was mixed with 1 mL of complete Freund's adjuvant (CFA) and injected subcutaneously for primary immunization. For the second immunization on day 21, 0.25 mg of human BCMA-hFc protein was mixed with 1 mL of incomplete Freund's adjuvant (IFA) and then injected subcutaneously. For the third immunization on day 42, 0.25 mg of each of human BCMA-hFc protein and cynomolgus BCMA (Met1-Ala53)-hFc protein (purchased from Aero, catalog number: BCA-C5253) was mixed with 1 mL of IFA and injected subcutaneously. Thereafter, booster immunity would be given every three weeks according to the method used in the third immunization.

Blood was collected 1 week after each booster immunization, and serum antibody titer and specificity were detected using ELISA. The results are shown in Table 6. This shows that the serum of alpacas immunized with human BCMA-hFc and cynomolgus monkey BCMA-hFc proteins can bind to the immunogen to varying degrees, showing an antigen-antibody reaction. The blank control was 1% (w/v) BSA, and the fourth and fifth immunizations referred to the alpaca serum on the seventh day after the fourth and fifth immunizations respectively. The data in the table are OD450 nm values, and k represents 1000.

**Table 6 ELISA detection of antibody titer in alpaca serum after protein immunization**

| **Serum dilution** | **The fourth immunizatio n** | **The fifth immunizatio n** | **The fourth immunizatio n** | **The fifth immunizatio n** |
|---|---|---|---|---|
| | **huBCMA-His** | | **CynoBCMA-his** | |
| 1 : 16k | 3.95 | 3.85 | 1.71 | 1.72 |
| 1 : 32k | 3.64 | 3.56 | 1.42 | 1.22 |
| 1 : 64k | 3.12 | 2.67 | 0.90 | 0.66 |
| 1 : 128k | 2.26 | 1.90 | 0.42 | 0.36 |
| 1 : 256k | 1.15 | 0.92 | 0.22 | 0.21 |
| 1 : 512k | 0.79 | 0.62 | 0.18 | 0.15 |
| 1 : 1024k | 0.41 | 0.33 | 0.08 | 0.09 |
| Blank control | 0.04 | 0.05 | 0.04 | 0.04 |

### (B) Phage library construction

PBMC were separated from 50 mL of peripheral blood collected after the fourth and fifth immunizations according to the instructions for using the lymphocyte separation solution. RNAiso Plus reagent was used to extract total RNA of PBMC obtained from the NB254 subjected to the fourth and fifth immunizations. PrimeScript^{™}II 1st Strand cDNA Synthesis Kit (Takara, catalog number: 6210A) was used for reverse transcription, and cDNA was prepared by reverse transcription according to the instructions, and a total of 5 µg RNA was transcribed. The cDNA stock solutions from the fourth and fifth immunizations were mixed in equal proportions and then the mixture was diluted 5-fold for nested amplification, and then the gel was cut to recover the VHH fragment. The vector and the target fragment were digested with SfiI overnight at 50°C and recovered. The vector and the target fragment were ligated at a ratio of 1 : 3. 10 electroporation transformations were performed on the ligation product. Immediately after electroporation, 1 mL of 2YT medium (preheated at 37°C) was added to the electroporation cuvette for recovery. The electroporation product was aspirated out and the electroporation cuvette was washed with 2YT culture medium. Recovery was performed at 37°C and 180 rpm for 45 min. 100 µL was taken and gradiently diluted to 10⁻³ and 10⁻⁴ to determine the number of transformants in the library, and spread on a 90 mm plate. The rest was centrifuged, 8 mL of 2YT culture medium was added for resuspension and the suspension was spread on eight 200 mm plates. Counting was performed on the next day to calculate storage capacity as 2.14 × 10⁹. 48 clones were randomly selected and cultured, and the insertion rate was 100% by PCR.

*E.coli* library was inoculated into 2 × 300 mL 2YT (Amp: 100 µg/ml, Glu: 1%) culture medium until its initial OD600 = 0.1-0.2, and cultured at 37°C and 230 rpm until OD600 = 0.8 or above. The amount of helper phage M13K07 added was calculated based on OD600 (M13K07:E. coli = 20 : 1), the cells were left to be infected at rest at 37°C for 30 min and then the infected cells were cultured at 37°C and 180 rpm for 30 min. The supernatant was removed by centrifugation at 5000 rpm, the medium was replaced with 2YT (100 µg/mL Amp + 50 µg/mL Kan) medium and culture was performed at 30°C and 220 rpm overnight. The phage library was precipitated according to the procedure on NEB official website and the titer was determined.

### (C) Panning of nanobody against BCMA

The microplate was coated with human BCMA-hFc protein at 0.5 µg/well, with a total of 8 wells. The input amount of the phage library was 2.2 × 10¹¹, and the measured titer of the elution product in first round was 1.85 × 10⁷. After identification by ELISA, the product of the first round panning has a positive rate of 89.58% for the binding to human BCMA protein, a positive rate of 4.2% for binding to monkey BCMA protein, and a positive rate of 4.2% for cross-binding to human and monkey BCMA proteins. The elution product of the first round panning was amplified according to the conventional procedure, and the titer of the amplified phage was determined to be 1.14 × 10¹³. The microplate for the second round panning was coated with monkey BCMA protein at 0.2 µg/well, with a total of 4 wells; The input amount of phage library was 2.2 × 10¹¹, and the titer of the elution product was determined to be 1.32 × 10⁷ After identification by ELISA, the product of the second round panning has a positive rate of 92.1% for the binding to human BCMA protein, a positive rate of 39.3% for binding to monkey BCMA protein, and a positive rate of 39.3% for cross-binding to human and monkey BCMA proteins.

### (D) Sequencing

After panning, a total of 70 human-monkey cross positive clones were obtained. The positive clones were sent for sequencing analysis. A phylogenetic tree was constructed based on the VHH encoding protein sequence. After cluster analysis, it was found that the 70 sequences belonged to 3 lines, and a total of 8 clones (Lab01, 02, 03, 04, 05, 06, 07 and 08) were selected for construction, expression and identification, respectively.

### Example 3 Construction and expression of recombinant candidate antibodies

The VHH sequence of interest was recombined into the expression vector of human IgG1 Fc to obtain a recombinant plasmid (see SEQ ID NO: 4 for the sequence of human IgG1 Fc). The recombinant candidate antibody plasmid and transfection reagent PEI (purchased from Polysciences, catalog number: 24765-1) were added to OPTI-MEM (Gibco, catalog number: 11058021), mixed well, let stand for 15 min, and then added to Expi293F cells (Manufacturer: Thermofisher, catalog number: A14527), the obtained mixture was put in a shaker for culture at 5% CO₂, 120 rpm and 37°C. On the second day of transfection, OPM-293 ProFeed (Shanghai OPM Biosciences Co., Ltd., catalog number: F081918-001) and 6 g/L glucose (Manufacturer: Sigma, catalog number: G7528) were added. On the sixth day of transfection, the cell supernatant was collected. The culture supernatant was collected and purified by affinity chromatography with Protein A. The packing used was Mabselect SuRe (No. 10054120, purchased from GE Healthcare). The buffer was Mab affinity balance solution (PBS Buffer) and Mab affinity elution liquid (containing 1M NaCl in PBS buffer) and Mab affinity eluent (citrate buffer). Concentration was determined with an ultramicrovolume spectrophotometer (Nanodrop8000, Thermo). Protein samples to be detected were analyzed by SEC-HPLC to characterize the molecular size homogeneity and purity of the proteins. The HPLC used was Agilent 1260, the chromatographic column TSKgel G3000SWXI, was from Tosoh Bioscience, the mobile phase was 200 mM phosphate buffer (pH 7.0)/isopropanol (v/v 9 : 1) (batch number: 20210519001), the detection temperature was 25°C, the flow rate was 0.5 mL/min, and the detection wavelength was 280 nm. For the SEC-HPLC data, the chromatogram was analyzed by a manual integration method, and the protein purity was calculated by an area normalization method. The main peak was considered as a monomer, the chromatographic peak before the main peak was called an aggregate, and the chromatographic peak after the main peak was called a fragment. The results are shown in Table 7. The final product of the candidate antibody had high purity.

**Table 7 Results of expression quantity and SEC purity of BCMA VHH-hFc candidate antibody**

| **Designation of antibody** | **Expressio n system** | **Expressi on volume (ml)** | **Concent ration (mg/ml)** | **Volume (ml)** | **SEC monomer (%)** |
|---|---|---|---|---|---|
| BCMA-Lab01 | expi 293F | 40 | 2.46 | 2 | 98.43 |
| BCMA-Lab05 | expi 293F | 40 | 3.01 | 2 | 96.04 |
| BCMA-Lab06 | expi 293F | 40 | 2.31 | 2 | 98.15 |
| BCMA-Lab07 | expi 293F | 40 | 6.91 | 2 | 97.91 |
| BCMA-Lab04 | expi 293F | 40 | 2.15 | 2 | 97.59 |
| BCMA-Lab08 | expi 293F | 40 | 2.45 | 2 | 98.48 |
| BCMA-Lab03 | expi 293F | 40 | 1.71 | 2 | 98.81 |
| BCMA-Lab02 | expi 293F | 40 | 3.47 | 2 | 97.6 |

### Example 4 Identification of recombinant candidate antibodies

### (A) Detection of binding of BCMA recombinant candidate antibodies to human BCMA protein and cross-binding of BCMA recombinant candidate antibodies to monkey BCMA protein by enzyme-linked immunosorbent assay (ELISA)

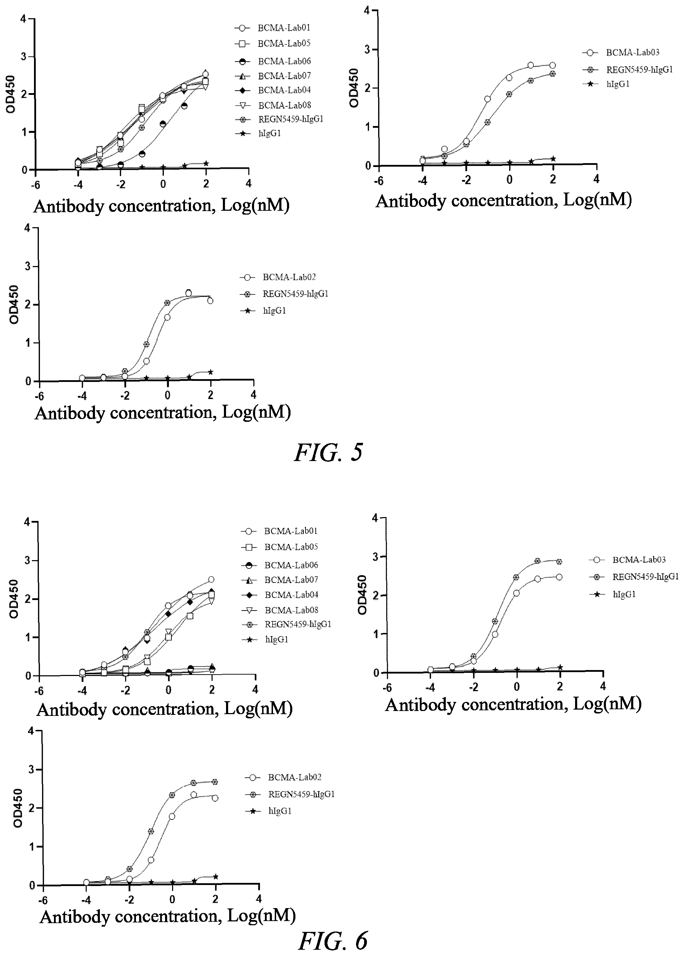

The specific method was as follows: Human BCMA protein (purchased from Aero, catalog number: BCA-H522y) and monkey BCMA protein (purchased from Aero, catalog number: BCA-C52H7) were diluted with PBS to a final concentration of 1 µg/mL, respectively, and then added to a 96-well ELISA plate at 50 µl/well. The plate was sealed with a plastic film and incubated overnight at 4°C, the plate was washed twice with PBS the next day, and then a blocking solution [PBS+2% (w/v) BSA] was added for blocking at room temperature for 2 h. The blocking solution was discarded, the plate was washed 3 times with PBS, and 100 nM BCMA recombinant chimeric nanobody diluted gradiently or negative control antibody was added at 50 µl/well. After incubation at 37°C for 2 h, the plate was washed 3 times with PBS. HRP (horseradish peroxidase)-labeled secondary antibody (purchased from Merck, catalog number: AP113P) was added, and incubated at 37°C for 1 h, and the plate was washed 5 times with PBS. TMB substrate was added at 50 µl/well, and incubated at room temperature for 10 min, then a stop solution (1.0 M HCl) was added at 50 µl/well. The OD450 nm values were read by an ELISA microplate reader (Multimode Plate Reader, EnSight, purchased from Perkin Elmer). The results are shown in Fig. 5 and Fig. 6. All antibodies bound to human BCMA protein. BCMA-Lab06 and BCMA-Lab07 had no cross-binding activity with monkey BCMA protein. All other recombinant candidate antibodies had cross-binding activity with monkey BCMA protein. The IgG isotype control was human IgG1.

### (B) Detection of binding of BCMA recombinant candidate antibodies to endogenous cells expressing BCMA by flow cytometry experiment (FACS)

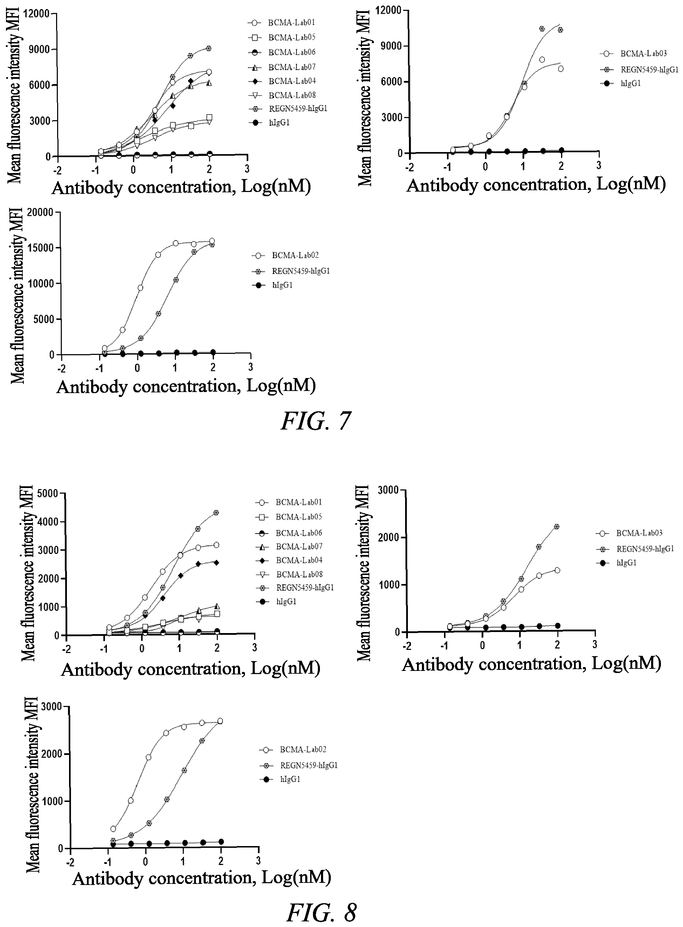

The desired cells were scale-up cultured in T-75 cell culture flasks to the logarithmic growth phase, and the cells were pipetted into a single cell suspension. After the cells were counted, the cells were centrifuged and the cell pellet was resuspended in FACS buffer (PBS + 2% fetal calf serum) to 2 × 10⁶ cells per ml, and the suspension was added to the 96-well FACS reaction plate at 50 µl per well, then the sample to be tested of the candidate antibody was added at 50 µl per well and the mixture was incubated at 4°C for 1 h. The obtained mixture was centrifuged and washed 3 times with a PBS buffer. Secondary antibody anti-hIgG(Fc) Alexa 647 (purchased from Jackson, catalog number: 109-605-098) was added at 50 µl/well, and incubated on ice for 1 h. The obtained mixture was centrifuged and washed 3 times with a PBS buffer. 100 µl of the mixture was taken for detection and result analysis by FACS (FACS CantoTM, purchased from BD Company). Data analysis was performed by software (FlowJo) to obtain the mean fluorescence intensity (MFI) of the cells. Then, a software (GraphPad Prism8) was used for analysis, data fitting and EC50 calculation. The results are shown in Table 8 and Figs. 7 and 8. BCMA-Lab05, BCMA-Lab06, and BCMA-Lab08 had poor binding to H929 and U266 cells, while the other antibodies performed better.

**Table 8 FACS detection of binding activity of recombinant candidate antibodies with H929 and U266 cells**

| **Designation of antibody** | **H929** | | **U266** | |
|---|---|---|---|---|
| | **Maximum mean fluorescence intensity** | **EC50 (nM)** | **Maximum mean fluorescence intensity** | **EC50 (nM)** |
| BCMA-Lab01 | 6983 | 2.82 | 3137 | 1.80 |
| BCMA-Lab05 | 3186 | 1.42 | 714 | 3.63 |
| BCMA-Lab06 | 125 | Negative | 75 | Negative |
| BCMA-Lab07 | 6081 | 2.85 | 985 | 10.80 |
| BCMA-Lab04 | 6897 | 7.72 | 2512 | 3.93 |
| BCMA-Lab08 | 2772 | 3.34 | 693 | 5.14 |
| REGN5459-hIgG1 | 8957 | 5.24 | 4272 | 7.14 |
| hIgG1 | 99 | Negative | 108 | Negative |

| **Designation of antibody** | **H929** | | **U266** | |
|---|---|---|---|---|
| | **Maximum mean fluorescence intensity** | **EC50 (nM)** | **Maximum mean fluorescence intensity** | **EC50 (nM)** |
| BCMA-Lab03 | 7757 | 5.36 | 1280 | 7.05 |
| REGN5459-hIgG1 | 10349 | 9.39 | 2202 | 16.61 |
| hIgG1 | 91 | Negative | 111 | Negative |

| **Designation of antibody** | **H929** | | **U266** | |
|---|---|---|---|---|
| | **Maximum mean fluorescence intensity** | **EC50 (nM)** | **Maximum mean fluorescence intensity** | **EC50 (nM)** |
| BCMA-Lab02 | 15783 | 0.96 | 2671 | 0.62 |
| REGN5459-hIgG1 | 15256 | 6.46 | 2654 | 9.49 |
| hIgG1 | 167 | Negative | 119 | Negative |

### (C) Detection of blocking effect of recombinant candidate antibodies on the binding of ligand APRIL to human BCMA protein by ligand binding competition ELISA

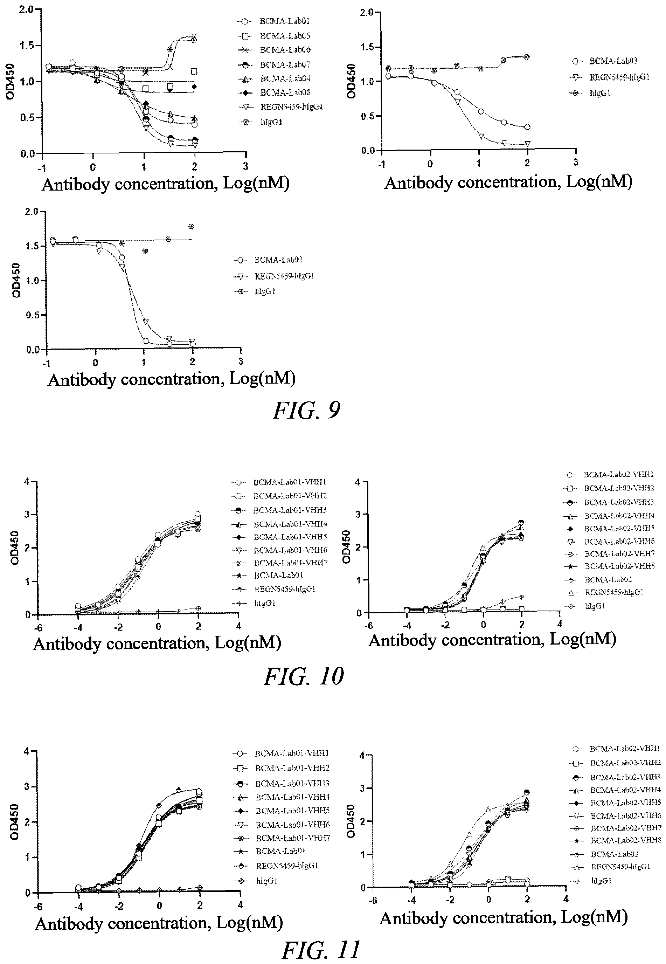

The antigen protein was diluted with PBS to a final concentration of 1 µg/mL, and then added to a 96-well ELISA plate at 50 µl per well. The plate was sealed with a plastic film and incubated overnight at 4°C, the plate was washed twice with PBS the next day, and then a blocking solution [PBS + 2% (w/v) BSA] was added for blocking at room temperature for 2 h. The blocking solution was discarded, the plate was washed 3 times with PBS, and 200 nM BCMA recombinant chimeric nanobody diluted gradiently or negative control antibody was added at 50 µl/well. hAPRIL-biotin (purchased from Aero, catalog number: APL-H82F5) was diluted to 0.5 µg/mL, with 50 µl per well. After incubation at 37°C for 1.5 h, the plate was washed 3 times with PBS. HRP (horseradish peroxidase)-labeled secondary antibody (purchased from Sigma, catalog number: S2438-250 µg) was added, and incubated at 37°C for 1 h, and the plate was washed 5 times with PBS. TMB substrate was added at 50 µl/well, and incubated at room temperature for 10 min, then a stop solution (1.0 M HCl) was added at 50 µl/well. The OD450 nm values were read by an ELISA microplate reader (Multimode Plate Reader, EnSight, purchased from Perkin Elmer). The results are shown in Fig. 9. BCMA-Lab05, BCMA-Lab06 and BCMA-Lab08 had average blocking effects, while the other antibodies had good blocking effects.

### Example 5 Detection of affinity of BCMA recombinant candidate antibodies

Anti-BCMA recombinant candidate antibodies were captured using a Protein A chip (GE Healthcare; 29-127-558). A sample buffer and a running buffer were HBS-EP + (10 mM HEPES, 150 mMNaCl, 3 mMEDTA and 0.05% surfactant P20) (GE Healthcare; BR-1006-69). The flow cell was set to 25°C. The sample block was set to 16°C. Both were pretreated with the running buffer. In each cycle, an antibody to be detected was first captured with a Protein A chip, and then a single concentration of BCMA antigen protein was injected. The binding and dissociation processes of the antibody and the antigen protein were recorded, and finally Glycine pH 1.5 (GE Healthcare; BR-1003-54) was used to complete chip regeneration. Binding was measured by injecting different concentrations of BCMA-His in solution for 240 s with a flow rate of 30 µL/min. The concentration started from 200 nM (see the detailed results for the actual concentration in the test) and was diluted at 1 : 1, making a total of 5 concentrations. The dissociation phase was monitored for up to 600 s and was triggered by switching from sample solution to running buffer. The surface was regenerated by washing with a 10 mM glycine solution (pH 1.5) for 30 s at a flow rate of 30 µL/min. Bulk refractive index difference was corrected by subtracting the response obtained from the goat anti-human Fc surface. Blank injection was also subtracted (= double reference). For calculation of apparent KD and other kinetic parameters, Langmuir 1 : 1 model was used. The binding rate (Kₐ), dissociation rate (K_{dis}) and binding affinity (KD) of the recombinant candidate antibody to human BCMA protein are shown in the table, wherein REGN5459-hIgG 1 antibody was used as a control. As shown in Table 9, the KD values of all the recombinant candidate antibodies for human BCMA protein are below 1E-7M.

**Table 9 Binding affinity of recombinant candidate antibodies to human BCMA protein**

| **Designation of antibody** | **Kₐ (1/Ms)** | **K_{dis} (1/s)** | **KD (M)** |
|---|---|---|---|
| BCMA-Lab01 | 1.33E + 06 | 7.02E-04 | 5.27E-10 |
| BCMA-Lab04 | 8.36E + 05 | 2.69E-03 | 3.22E-09 |
| BCMA-Lab08 | 1.32E + 07 | 2.75E-01 | 2.08E-08 |
| BCMA-Lab03 | 8.29E + 05 | 3.15E-03 | 3.79E-09 |
| BCMA-Lab02 | 6.88E + 05 | 4.93E-05 | 7.16E-11 |
| REGN5459-hIgG1 | 7.66E + 05 | 1.78E-04 | 2.33E-10 |

After a series of experimental screenings, BCMA-Lab01 and BCMA-Lab02 alpaca antibodies were selected for subsequent humanization engineering. The sequences of BCMA-Lab01 - BCMA-Lab08 alpaca antibodies are shown in Table 10, and the CDR sequences analyzed by Kabat, IMGT, and Chothia methods are shown in Table 11.

**Table 10 The amino acid sequences of BCMA alpaca antibodies**

| **Design ation of antibo dy** | **No.** | **Amino acid sequence** |
|---|---|---|
| BCMA -Lab01 | SEQ ID NO: 7 | QVQLVESGGGLVQPGGSLRLSCSGSGFTLDYYMIGWFRQAPGKER EGLSSISPADGSTYYADSVKGRFTISRDSAKNTVYLQMNNLKPEDT ALYYCAAGNEATISWGFGPWEYDYWGQGTQVTVSS |
| BCMA -Lab02 | SEQ ID NO: 8 | QVQLVESGGGLVQSRGSLRLSCAASESISSIHIMAWYRRAPGKQRE LVAGIRNDGSTVYADSAKGRFTISRDNAKNTVYLQMNSLKPEDTA VYYCNADQGFGSYSEWERRSRWGQGTEVTVSS |
| BCMA -Lab03 | SEQ ID NO: 9 | EVQVVESGGGLVQPGGSLRLSCAGSGFTLAHYMIGWFRQAPGKER EGLSSISPADGSTYYADSVKGRFTISRDNAKNTVYLQMNSLKPEDT ALYYCAAGNEATISWGFGPYEYDYWGQGTQVTVSS |
| BCMA -Lab04 | SEQ ID NO: 10 | EVQVVESGGGLVQPGGSLRLSCAGSGFTLAYYMIGWFRQAPGKER EGLSSISPADGSTYYADSVKGRFTISRDNAKNTVYLQMNSLKPEDT ALYYCAAGNEAAITWGFGPYEYDYWGQGTQVTVSS |
| BCMA -Lab05 | SEQ ID NO: 11 | QVQLVESGGGLVQPGGSLRLSCAASGFTLAYYMIGWFRQAPGKER EGVSSISSGTGSTYYANSVKDRFTISRDNAANKVYLEMNSLKPEDT AIYYCAAGNEATITWGFGPYEYDYWGQGTQVTVSS |
| BCMA -Lab06 | SEQ ID NO: 12 | EVQVVESGGGLVQPGGSLRLSCAASGFTLAYYGIGWFRQAPGQER EGVASITASDGSTYYADSVKDRFTISRDNAANTVYLQMNSLKPEDT AVYFCAAGNDGAISWGFGPYEYDYWGQGTQVTVSS |
| BCMA -Lab07 | SEQ ID NO: 13 | QVQLVESGGGLVQPGGSLRLSCAASGFTFRSSAMSWARQAPGKGL EWVSAIYPDGRTYYADSIKGRFTSSRDNAKNTVYLQMNSLRSEDT AIYYCTIGNQLSDLPLGQGTQVTVSS |
| BCMA -Lab08 | SEQ ID NO: 14 | QVQLVESGGGLVQPGGSLRLSCTDSGFTLNYYMIGWFRQAPGKEQ EGLSSISAGDGSTYYADSVKGRFTISRDYAKNTVYLEMNDLKPEDT GVYYCAAGNEGAITWGFGPYEYDYWGQGTQVTVSS |

**Table 11 Analysis of CDR sequences by Kabat, IMGT and Chothia numbering**

| **Desi gnat ion of anti bod y** | **Anal ysis meth od** | **CDR1** | **CDR2** | **CDR3** |
|---|---|---|---|---|
| BC MA Lab 01 | Kaba t | YYMIG (SEQ ID NO: 34) | SISPADGSTYYADSVK G (SEQ ID NO: 35) | GNEATISWGFGPWEYDY (SEQ ID NO: 36) |
| | IMG T | GFTLDYYM (SEQ ID NO: 37) | ISPADGST (SEQ ID NO: 38) | AAGNEATISWGFGPWEY DY (SEQ ID NO: 39) |
| | Chot hia | GFTLDYY (SEQ ID NO: 40) | SPADGS (SEQ ID NO: 41) | GNEATISWGFGPWEYDY (SEQ ID NO: 36) |
| BC MA | Kaba t | IHIMA (SEQ ID NO: 42) | GIRNDGSTVYADSAKG (SEQ ID NO: 43) | DQGFGSYSEWERRSR (SEQ ID NO: 44) |
| - Lab 02 | IMG T | ESISSIHI (SEQ ID NO: 45) | IRNDGST (SEQ ID NO: 46) | NADQGFGSYSEWERRSR (SEQ ID NO: 47) |
| | Chot hia | ESISSIH (SEQ ID NO: 48) | RNDGS (SEQ ID NO: 49) | DQGFGSYSEWERRSR (SEQ ID NO: 44) |
| BC MA Lab 03 | Kaba t | HYMIG (SEQ ID NO: 50) | SISPADGSTYYADSVK G (SEQ ID NO: 35) | GNEATISWGFGPYEYDY (SEQ ID NO: 51) |
| | IMG T | GFTLAHYM (SEQ ID NO: 52) | ISPADGST (SEQ ID NO: 38) | AAGNEATISWGFGPYEY DY (SEQ ID NO: 53) |
| | Chot hia | GFTLAHY (SEQ ID NO: 54) | SPADGS (SEQ ID NO: 41) | GNEATISWGFGPYEYDY (SEQ ID NO: 51) |
| BC MA Lab 04 | Kaba t | YYMIG (SEQ ID NO: 34) | SISPADGSTYYADSVK G (SEQ ID NO: 35) | GNEAAITWGFGPYEYDY (SEQ ID NO: 55) |
| | IMG T | GFTLAYYM (SEQ ID NO: 56) | ISPADGST (SEQ ID NO: 38) | AAGNEAAITWGFGPYEY DY (SEQ ID NO: 57) |
| | Chot hia | GFTLAYY (SEQ ID NO: 58) | SPADGS (SEQ ID NO: 41) | GNEAAITWGFGPYEYDY (SEQ ID NO: 55) |
| BC MA Lab 05 | Kaba t | YYMIG (SEQ ID NO: 34) | SISSGTGSTYYANSVK D (SEQ ID NO: 59) | GNEATITWGFGPYEYDY (SEQ ID NO: 60) |
| | IMG T | GFTLAYYM (SEQ ID NO: 56) | ISSGTGST (SEQ ID NO: 61) | AAGNEATITWGFGPYEY DY (SEQ ID NO: 62) |
| | Chot hia | GFTLAYY (SEQ ID NO: 58) | SSGTGS (SEQ ID NO: 63) | GNEATITWGFGPYEYDY (SEQ ID NO: 60) |
| BC MA Lab 06 | Kaba t | YYGIG (SEQ ID NO: 64) | SITASDGSTYYADSVK D (SEQ ID NO: 65) | GNDGAISWGFGPYEYDY (SEQ ID NO: 66) |
| | IMG T | GFTLAYYG (SEQ ID NO: 67) | ITASDGST (SEQ ID NO: 68) | AAGNDGAISWGFGPYEY DY (SEQ ID NO: 69) |
| | Chot hia | GFTLAYY (SEQ ID NO: 58) | TASDGS (SEQ ID NO: 70) | GNDGAISWGFGPYEYDY (SEQ ID NO: 66) |
| BC MA Lab 07 | Kaba t | SSAMS (SEQ ID NO: 71) | AIYPDGRTYYADSIKG (SEQ ID NO: 72) | GNQLSDLP (SEQ ID NO: 73) |
| | IMG T | GFTFRSSA (SEQ ID NO: 74) | IYPDGRT (SEQ ID NO: 75) | TIGNQLSDLP (SEQ ID NO: 76) |
| | Chot hia | GFTFRSS (SEQ ID NO: 77) | YPDGR (SEQ ID NO: 78) | GNQLSDLP (SEQ ID NO: 73) |
| BC MA | Kaba t | YYMIG (SEQ ID NO: 34) | SISAGDGSTYYADSVK G (SEQ ID NO: 79) | GNEGAITWGFGPYEYDY (SEQ ID NO: 80) |
| Lab 08 | IMG T | GFTLNYYM (SEQ ID NO: 81) | ISAGDGST (SEQ ID NO: 82) | AAGNEGAITWGFGPYEY DY (SEQ ID NO: 83) |
| | Chot hia | GFTLNYY (SEQ ID NO: 84) | SAGDGS (SEQ ID NO: 85) | GNEGAITWGFGPYEYDY (SEQ ID NO: 80) |

### Example 6 Humanization of Nanobodies

By aligning the IMGT (http://imgt.cines.fr) human antibody heavy and light chain variable region germline gene database, germline genes of heavy chain variable region having high homology to the VHH nanobody were selected as templates, respectively, and the CDRs of the VHH nanobody were grafted into the corresponding human template, to form a variable region sequence in the order of FR1-CDR1-FR2-CDR2-FR3-CDR3-FR4. According to needs, the key amino acids in the framework sequence were back-mutated to the corresponding amino acids of the VHH nanobody to ensure the original affinity, and thus the humanized monoclonal antibody was obtained. The CDR amino acid residues of the antibody were determined and annotated by the Kabat numbering system.

### (A) Humanization of BCMA-Lab01 antibody

The humanization heavy chain templates of VHH nanobody BCMA-Lab01 are IGHV3-64*04 and IGHJ3*01. The CDRs of VHH nanobody BCMA-Lab01 were grafted into the human templates of the nanobody respectively, and thus the corresponding humanized versions were obtained. According to needs, the key amino acids in the FR region sequence of the humanized antibody of BCMA-Lab01 were back-mutated to the corresponding amino acids of the VHH nanobody to ensure the original affinity. There were sites prone to chemical modification in the antibody, and point mutations at these sites had eliminated the risk of modification. The specific mutation designs are shown in Table 12.

**Table 12 Mutation design of humanized antibody of BCMA-Lab01**

| **VHH** | |
|---|---|
| H1 | Graft(IGHV3-64*04) + H35G,V37F,G44E,L45R,Y47G,A50S |
| H2 | Graft(IGHV3-64*04) + H35G,V37F,G44E,L45R,Y47G,A50S,N74S |
| H3 | Graft(IGHV3-64*04) + M34I,H35G,V37F,G44E,L45R,Y47G,A50S,N74S,L79V |
| H4 | Graft(IGHV3-64*04) + M34I,H35G,V37F,G44E,L45R,Y47G,A50S,N74S,S75A,L79V |
| H5 | Graft(IGHV3-64*04) + M34I,H35G,V37F,G44E,L45R,Y47G,A50S,N74S,L79V,V93L,M121Q |
| H6 | Graft(IGHV3-64*04) + M34I,H35G,V37F,G44E,L45R,Y47G,V48L,A50S,N74S,L79V |
| H7 | Graft(IGHV3-64*04) + M34I,H35G,V37F,G44E,L45R,Y47G,A50S,N74S,L79V + E1Q |

| | |
|---|---|
| Notes: Graft represents the grafting of the CDRs of the VHH nanobody into the FR region sequence of the human germline template; H35G represents the mutation of H at position 35 of Graft into G, and so on. The numbering of backmutated amino acids is according to the natural sequence numbering. | |

The specific sequence of the variable region of the humanized antibody of BCMA-Lab01 is as follows:
The amino acid sequence of BCMA-Lab01-VHH1 is as shown in SEQ ID NO: 15: 
EVQLVESGGGLVQPGGSLRLSCSASGFTLDYYMMGWFRQAPGKEREGVSSISPADGST
YYADSVKGRFTISRDNSKNTLYLQMNSLRAEDTAVYYCAAGNEATISWGFGPWEYDYWG
QGTMVTVSS
The amino acid sequence of BCMA-Lab01-VHH2 is as shown in SEQ ID NO: 16: 
EVQLVESGGGLVQPGGSLRLSCSASGFTLDYYMMGWFRQAPGKEREGVSSISPADGST
YYADSVKGRFTISRDSSKNTLYLQMNSLRAEDTAVYYCAAGNEATISWGFGPWEYDYWGQ
GTMVTVSS
The amino acid sequence of BCMA-Lab01-VHH3 is as shown in SEQ ID NO: 17: 
EVQLVESGGGLVQPGGSLRLSCSASGFTLDYYMIGWFRQAPGKEREGVSSISPADGST
YYADSVKGRFTISRDSSKNTVYLQMNSLRAEDTAVYYCAAGNEATISWGFGPWEYDYWG
QGTMVTVSS
The amino acid sequence of BCMA-Lab01-VHH4 is as shown in SEQ ID NO: 18: 
EVQLVESGGGLVQPGGSLRLSCSASGFTLDYYMIGWFRQAPGKEREGVSSISPADGST
YYADSVKGRFTISRDSAKNTVYLQMNSLRAEDTAVYYCAAGNEATISWGFGPWEYDYWG
QGTMVTVSS
The amino acid sequence of BCMA-Lab01-VHHS is as shown in SEQ ID NO: 19: 
EVQLVESGGGLVQPGGSLRLSCSASGFTLDYYMIGWFRQAPGKEREGVSSISPADGST
YYADSVKGRFTISRDSSKNTVYLQMNSLRAEDTALYYCAAGNEATISWGFGPWEYDYWGQ
GTQVTVSS
The amino acid sequence of BCMA-Lab01-VHH6 is as shown in SEQ ID NO: 20: 
EVQLVESGGGLVQPGGSLRLSCSASGFTLDYYMIGWFRQAPGKEREGLSSISPADGSTY
YADSVKGRFTISRDSSKNTVYLQMNSLRAEDTAVYYCAAGNEATISWGFGPWEYDYWGQ
GTMVTVSS
The amino acid sequence of BCMA-Lab01-VHH7 is as shown in SEQ ID NO: 21: 
QVQLVESGGGLVQPGGSLRLSCSASGFTLDYYMIGWFRQAPGKEREGVSSISPADGST
YYADSVKGRFTISRDSSKNTVYLQMNSLRAEDTAVYYCAAGNEATISWGFGPWEYDYWG
QGTMVTVSS
The amino acid sequence of humanization heavy chain template IGHV3-64*04 is as shown in SEQ ID NO: 22: 
QVQLVESGGGLVQPGGSLRLSCSASGFTFSSYAMHWVRQAPGKGLEYVSAISSNGGST
YYADSVKGRFTISRDNSKNTLYLQMNSLRAEDTAVYYCAR
The amino acid sequence of humanization heavy chain template IGHJ3*01 is as shown in SEQ ID NO: 23:
   WGQGTMVTVSS

According to the Kabat numbering system, the analysis results of the VHH sequence of the above-mentioned humanized nanobody are shown in Table 13.

**Table 13 Kabat analysis results of the VHH sequence of the humanized nanobody of BCMA-Lab01**

| **Variable region** | **CDR1** | **CDR2** | **CDR3** |
|---|---|---|---|
| BCMA-Lab01-VHH 1 | YYMMG (SEQ ID NO: 86) | SISPADGSTYYADSVKG (SEQ ID NO: 35) | GNEATISWGFGPWEYDY (SEQ ID NO: 36) |
| BCMA-Lab01-VHH2 | | | |
| BCMA-Lab01-VHH3 | YYMIG (SEQ ID NO: 34) | SISPADGSTYYADSVKG (SEQ ID NO: 35) | GNEATISWGFGPWEYDY (SEQ ID NO: 36) |
| BCMA-Lab01-VHH4 | | | |
| BCMA-Lab01-VHH5 | | | |
| BCMA-Lab01-VHH6 BCMA-Lab01-VHH7 | | | |

### (B) Humanization of BCMA-Lab02 antibody

The humanization heavy chain templates of VHH single domain antibody BCMA-Lab02 are IGHV3-7*01 and IGHJ6*01. The CDRs of VHH single domain antibody BCMA-Lab02 were grafted into the human templates of the nanobody respectively, and thus the corresponding humanized versions were obtained. According to needs, the key amino acids in the FR region sequence of the humanized antibody of BCMA-Lab02 were back-mutated to the corresponding amino acids of the VHH single domain antibody to ensure the original affinity. There were sites prone to chemical modification in the antibody, and point mutations at these sites had eliminated the risk of modification. The specific mutation designs are shown in Table 14.

**Table 14 Mutation design of humanized antibody of BCMA-Lab02**

| **VH** | |
|---|---|
| H1 | Graft(IGHV3-7*01) + V37Y,G44Q,L45R,W47L |
| H2 | Graft(IGHV3-7*01) + S35A,V37Y,G44Q,L45R,W47L |
| H3 | Graft(IGHV3-7*01) + S35A,V37Y,G44Q,L45R,W47L,N50G |
| H4 | Graft(IGHV3-7*01) + S35A,V37Y,G44Q,L45R,W47L,N50G,Y58V |
| H5 | Graft(IGHV3-7*01) + S35A,V37Y,Q39R,G44Q,L45R,W47L,N50G,Y58V |
| H6 | Graft(IGHV3-7*01) + S35A,V37Y,G44Q,L45R,W47L,N50G,Y58V,V60A |
| H7 | Graft(IGHV3-7*01) + S35A,V37Y,G44Q,L45R,W47L,N50G,Y58V,L78V |
| H8 | Graft(IGHV3-7*01) + S35A,V37Y,Q39R,G44Q,L45R,W47L,N50G,Y58V,T118E |

| | |
|---|---|
| Notes: Graft represents the grafting of the CDRs of the VHH single domain antibody into the FR region sequence of the human germline template; W47L represents the mutation of W at position 47 of Graft into L, and so on. The numbering of backmutated amino acids is according to the natural sequence numbering. | |

The specific sequence of the variable region of the humanized antibody of BCMA-Lab02 is as follows:
The amino acid sequence of BCMA-Lab02-VHH1 is as shown in SEQ ID NO: 24: 
EVQLVESGGGLVQPGGSLRLSCAASESISSIHIMSWYRQAPGKQRELVANIRNDGSTYY
VDSVKGRFTISRDNAKNSLYLQMNSLRAEDTAVYYCNADQGFGSYSEWERRSRWGQGTT
VTVSS
The amino acid sequence of BCMA-Lab02-VHH2 is as shown in SEQ ID NO: 25: 
EVQLVESGGGLVQPGGSLRLSCAASESISSIHIMAWYRQAPGKQRELVANIRNDGSTYY
VDSVKGRFTISRDNAKNSLYLQMNSLRAEDTAVYYCNADQGFGSYSEWERRSRWGQGTT
VTVSS
The amino acid sequence of BCMA-Lab02-VHH3 is as shown in SEQ ID NO: 26: 
EVQLVESGGGLVQPGGSLRLSCAASESISSIHIMAWYRQAPGKQRELVAGIRNDGSTYY
VDSVKGRFTISRDNAKNSLYLQMNSLRAEDTAVYYCNADQGFGSYSEWERRSRWGQGTT
VTVSS
The amino acid sequence of BCMA-Lab02-VHH4 is as shown in SEQ ID NO: 27: 
EVQLVESGGGLVQPGGSLRLSCAASESISSIHIMAWYRQAPGKQRELVAGIRNDGSTVY
VDSVKGRFTISRDNAKNSLYLQMNSLRAEDTAVYYCNADQGFGSYSEWERRSRWGQGTT
VTVSS
The amino acid sequence of BCMA-Lab02-VHHS is as shown in SEQ ID NO: 28: 
EVQLVESGGGLVQPGGSLRLSCAASESISSIHIMAWYRRAPGKQRELVAGIRNDGSTVY
VDSVKGRFTISRDNAKNSLYLQMNSLRAEDTAVYYCNADQGFGSYSEWERRSRWGQGTT
VTVSS
The amino acid sequence of BCMA-Lab02-VHH6 is as shown in SEQ ID NO: 29: 
EVQLVESGGGLVQPGGSLRLSCAASESISSIHIMAWYRQAPGKQRELVAGIRNDGSTVY
ADSVKGRFTISRDNAKNSLYLQMNSLRAEDTAVYYCNADQGFGSYSEWERRSRWGQGTT
VTVSS
The amino acid sequence of BCMA-Lab02-VHH7 is as shown in SEQ ID NO: 30: 
EVQLVESGGGLVQPGGSLRLSCAASESISSIHIMAWYRQAPGKQRELVAGIRNDGSTVY
VDSVKGRFTISRDNAKNSVYLQMNSLRAEDTAVYYCNADQGFGSYSEWERRSRWGQGTT
VTVSS
The amino acid sequence of BCMA-Lab02-VHH8 is as shown in SEQ ID NO: 31: 
EVQLVESGGGLVQPGGSLRLSCAASESISSIHIMAWYRRAPGKQRELVAGIRNDGSTVY
VDSVKGRFTISRDNAKNSLYLQMNSLRAEDTAVYYCNADQGFGSYSEWERRSRWGQGTE
VTVSS
The amino acid sequence of humanization heavy chain template IGHV3-7*01 is as shown in SEQ ID NO: 32: 
EVQLVESGGGLVQPGGSLRLSCAASGFTFSSYWMSWVRQAPGKGLEWVANIKQDGSE
KYYVDSVKGRFTISRDNAKNSLYLQMNSLRAEDTAVYYCAR
The amino acid sequence of humanization heavy chain template IGHJ6*01 is as shown in SEQ ID NO: 33:
   WGQGTTVTVSS

According to the Kabat numbering system, the analysis results of the VHH sequence of the above-mentioned humanized single domain antibody are shown in Table 15.

**Table 15 Kabat analysis results of the VHH sequence of the humanized single domain antibody of BCMA-Lab02**

| **Variab le region** | **CDR1** | **CDR2** | **CDR3** |
|---|---|---|---|
| BCMA -Lab02-VHH 1 | IHIMS (SEQ ID NO: 87) | NIRNDGSTYYVDSVKG (SEQ ID NO: 88) | DQGFGSYSEWERRSR (SEQ ID NO: 44) |
| BCMA -Lab02-VHH2 | IHIMA (SEQ ID NO: 42) | NIRNDGSTYYVDSVKG (SEQ ID NO: 88) | DQGFGSYSEWERRSR (SEQ ID NO: 44) |
| BCMA -Lab02-VHH3 | IHIMA (SEQ ID NO: 42) | GIRNDGSTYYVDSVKG (SEQ ID NO: 89) | DQGFGSYSEWERRSR (SEQ ID NO: 44) |
| BCMA -Lab02-VHH4 | IHIMA (SEQ ID NO: 42) | GIRNDGSTVYVDSVKG (SEQ ID NO: 90) | DQGFGSYSEWERRSR (SEQ ID NO: 44) |
| BCMA -Lab02-VHH5 | | | |
| BCMA -Lab02-VHH7 | | | |
| BCMA -Lab02-VHH8 | | | |
| BCMA -Lab02-VHH6 | IHIMA (SEQ ID NO: 42) | GIRNDGSTVYADSVKG (SEQ ID NO: 91) | DQGFGSYSEWERRSR (SEQ ID NO: 44) |

### Example 7 Construction, expression and purity determination of humanized candidate nanobodies

The specific operating method referred to Example 3. The humanized VHH sequence of interest was recombined into the expression vector of human IgG1 Fc to obtain a recombinant plasmid (see SEQ ID NO: 4 for the sequence of human IgG1 Fc). The cell supernatant obtained by expressing the humanized VHH-Fc chimeric antibody was collected, and the purity of the obtained antibody was qualitatively analyzed by SEC-HPLC. The results are shown in Table 16, where "/" indicates that no detection was performed.

**Table 16 Results of expression quantity and SEC purity of BCMA humanized candidate nanobody**

| **Designation of antibody** | **Expressi on system** | **Expressi on volume (ml)** | **Concentrati on (mg/ml)** | **Volum e (ml)** | **SEC monom er** |
|---|---|---|---|---|---|
| BCMA-Lab01-VHH1 | expi 293F | 40 | 0.80 | 2 | 98.44 |
| BCMA-Lab01-VHH2 | expi 293F | 40 | 0.13 | 2 | 99.26 |
| BCMA-Lab01-VHH3 | expi 293F | 40 | 0.83 | 2 | 98.70 |
| BCMA-Lab01-VHH4 | expi 293F | 40 | 1.55 | 2 | 98.58 |
| BCMA-Lab01-VHH5 | expi 293F | 40 | 0.86 | 2 | 98.49 |
| BCMA-Lab01-VHH6 | expi 293F | 40 | 0.96 | 2 | 98.81 |
| BCMA-Lab01-VHH7 | expi 293F | 40 | 1.08 | 2 | 98.58 |
| BCMA-Lab02-VHH1 | expi 293F | 40 | 4.15 | 2 | / |
| BCMA-Lab02-VHH2 | expi 293F | 40 | 5.34 | 2 | / |
| BCMA-Lab02-VHH3 | expi 293F | 40 | 4.45 | 2 | / |
| BCMA-Lab02-VHH4 | expi 293F | 40 | 5.05 | 2 | / |
| BCMA-Lab02-VHH5 | expi 293F | 40 | 12.69 | 1.8 | 97.67 |
| BCMA-Lab02-VHH6 | expi 293F | 40 | 5.61 | 2 | / |
| BCMA-Lab02-VHH7 | expi 293F | 40 | 5.56 | 2 | 98.67 |
| BCMA-Lab02-VHH8 | expi 293F | 40 | 4.73 | 2 | / |

### Example 8 Identification of BCMA humanized candidate nanobodies

### (A) Detection of binding of BCMA humanized candidate nanobodies to human BCMA protein and cross-binding of BCMA humanized candidate nanobodies to monkey BCMA protein by enzyme-linked immunosorbent assay (ELISA)

The specific method was the same as in Example 4(A). The results are shown in Fig. 10 and Fig. 11. Both BCMA-Lab02-VHH1 and BCMA-Lab02-VHH2 did not bind to human BCMA and monkey BCMA proteins, and the other humanized candidate nanobodies had good binding with human BCMA, and had cross-binding activity with monkey BCMA protein. The IgG subtype control was human IgG1.

### (B) Detection of binding of humanized candidate nanobodies to endogenous cells expressing BCMA by flow cytometry experiment (FACS)

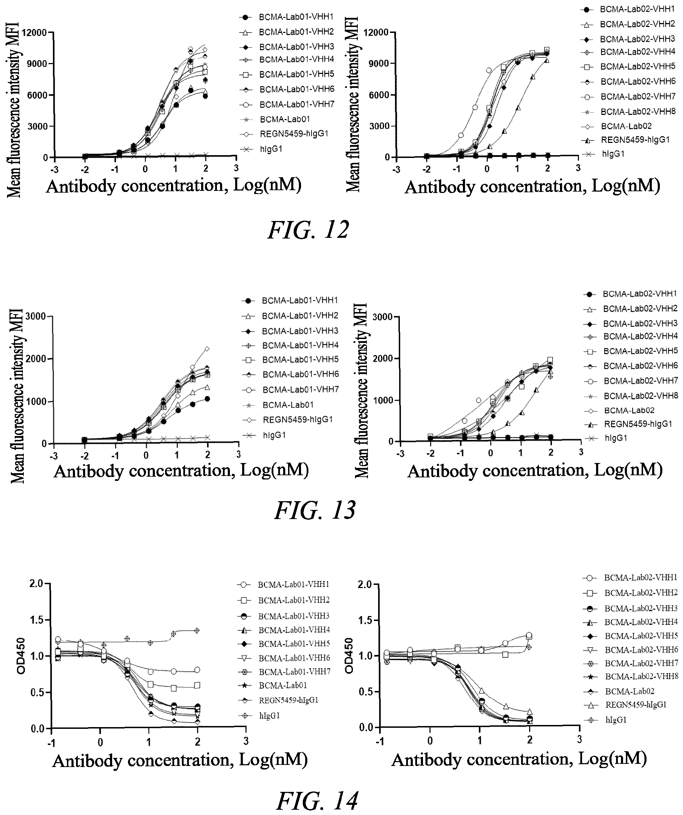

The specific method was the same as in Example 4(B). The results are shown in Table 17 and Figs. 12 and 13. BCMA-Lab02-VHH1 and BCMA-Lab02-VHH2 did not bind to H929 and U266 cells, and the other humanized candidate nanobodies had good binding with H929 and U266 cells.

**Table 17 FACS detection of the binding activity of humanized candidate nanobodies with H929 and U266 cells.**

| **Designation of antibody** | **H929** | | **U266** | |
|---|---|---|---|---|
| | **Maximum mean fluorescence intensity** | **EC50 (nM)** | **Maximu m mean fluoresc ence intensity** | **EC50 (nM)** |
| BCMA-Lab01-VHH1 | 6347 | 4.08 | 1031 | 5.43 |
| BCMA-Lab01-VHH2 | 6617 | 4.22 | 1304 | 6.75 |
| BCMA-Lab01-VHH3 | 9133 | 2.85 | 1659 | 3.48 |
| BCMA-Lab01-VHH4 | 8229 | 2.88 | 1608 | 3.99 |
| BCMA-Lab01-VHH5 | 9094 | 3.82 | 1591 | 4.01 |
| BCMA-Lab01-VHH6 | 10054 | 3.50 | 1753 | 3.25 |
| BCMA-Lab01-VHH7 | 8619 | 3.06 | 1596 | 3.71 |
| BCMA-Lab01 | 10017 | 3.05 | 1752 | 3.07 |
| REGN5459-hIgG1 | 10349 | 9.39 | 2202 | 16.61 |
| hIgG1 | 91 | Negative | 111 | Negative |
| BCMA-Lab02-VHH1 | 68 | Negative | 76 | Negative |
| BCMA-Lab02-VHH2 | 66 | Negative | 73 | Negative |
| BCMA-Lab02-VHH3 | 9830 | 2.27 | 1752 | 3.02 |
| BCMA-Lab02-VHH4 | 10061 | 1.45 | 1764 | 1.18 |
| BCMA-Lab02-VHH5 | 10219 | 1.33 | 1938 | 5.56 |
| BCMA-Lab02-VHH6 | 10141 | 1.54 | 1827 | 1.86 |
| BCMA-Lab02-VHH7 | 9842 | 0.39 | 1853 | 0.74 |
| BCMA-Lab02-VHH8 | 10151 | 1.49 | 1768 | 1.48 |
| BCMA-Lab02 | 10020 | 1.67 | 1840 | 1.45 |
| REGN5459-hIgG1 | 9206 | 13.16 | 1663 | 32.17 |
| hIgG1 | 121 | Negative | 124 | Negative |

### (C) Detection of blocking effect of humanized candidate nanobodies on the binding of ligand APRIL to human BCMA protein by ligand binding competition ELISA

The specific method was the same as in Example 4(C). The results are shown in Fig. 14. BCMA-Lab02-VHH1 and BCMA-Lab02-VHH2 cannot block the binding of ligand APRIL to human BCMA protein. BCMA-Lab01VHH1 and BCMA-Lab01VHH2 had poor blocking effects on the binding of ligand APRIL to human BCMA protein, while the other humanized candidate nanobodies had good blocking effects.

### Example 9 Detection of affinity of BCMA humanized candidate nanobodies

The specific operating method referred to Example 5. The binding rate (Ka), dissociation rate (Kdis) and binding affinity (KD) of the humanized candidate nanobodies to human BCMA protein are shown Table 18, wherein REGN5459-hIgG 1 antibody was used as a control. As shown in Table 18, the KD values of all the humanized candidate nanobodies for human BCMA protein are below 1E-8M.

**Table 18 Binding affinity of humanized candidate nanobodies to human BCMA protein**

| Designation of antibody | ka (1/Ms) | kd (1/s) | KD (M) |
|---|---|---|---|
| BCMA-Lab01-VHH1 | 1.19E + 06 | 8.41E-03 | 7.07E-09 |
| BCMA-Lab01-VHH2 | 1.04E + 06 | 3.61E-03 | 3.48E-09 |
| BCMA-Lab01-VHH3 | 1.15E + 06 | 1.38E-03 | 1.20E-09 |
| BCMA-Lab01-VHH4 | 1.14E + 06 | 1.37E-03 | 1.20E-09 |
| BCMA-Lab01-VHH5 | 1.20E + 06 | 1.41E-03 | 1.17E-09 |
| BCMA-Lab01-VHH6 | 1.31E + 06 | 8.02E-04 | 6.13E-10 |
| BCMA-Lab01-VHH7 | 1.11E + 06 | 1.47E-03 | 1.32E-09 |
| BCMA-Lab01 | 1.28E + 06 | 6.81E-04 | 5.33E-10 |
| BCMA-Lab02-VHH4 | 9.37E + 05 | 1.37E-04 | 1.46E-10 |
| BCMA-Lab02-VHH5 | 7.84E + 05 | 1.03E-04 | 1.32E-10 |
| BCMA-Lab02-VHH7 | 8.05E + 05 | 7.10E-05 | 8.82E-11 |
| BCMA-Lab02 | 7.38E + 05 | 4.47E-05 | 6.06E-11 |
| REGN5459-hIgG1 | 7.81E + 05 | 1.62E-04 | 2.08E-10 |

### Example 10 Panning of nanobody against BCMA and identification of recombinant candidate antibodies

### (A) Panning of nanobody against BCMA

According to the method of Example 2(C), positive clones that bound to human BCMA were further panned, and a total of 10 clones of Lab09 to 18 were selected for construction, expression, and identification.

### (B) Construction and expression of recombinant candidate antibodies

According to the method in Example 3, the recombinant candidate antibodies of VHH-IgG1 were obtained. At the same time, it was identified by SEC-HPLC that the purity of the final products of the candidate antibodies were relatively high.

### (C) ELISA detection of the binding of BCMA recombinant candidate antibodies to human BCMA protein

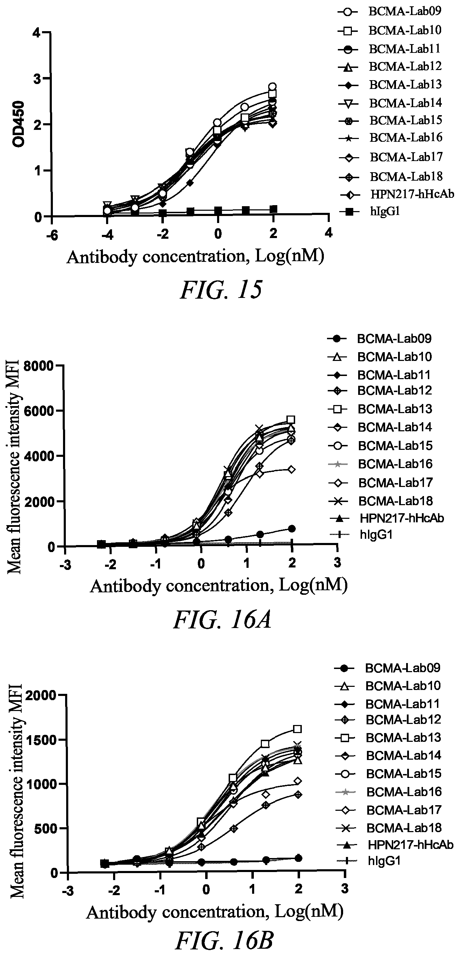

Referring to the method in Example 4(A), ELISA was used to detect the binding of the recombinant candidate antibodies to human BCMA. The results are shown in Fig. 15. Fig. 15 shows that all antibodies bound to human BCMA protein and were equivalent to the positive control antibody HPN217-hHcAb.

### (D) FACS detection of binding of BCMA recombinant candidate antibodies to endogenous cells expressing BCMA

According to the method in Example 4(B), FACS was used to identify the binding of the recombinant candidate antibodies to endogenous cells expressing BCMA, and the results are shown in Figs. 16A and 16B and Table 19. The results show that except for BCMA-Lab09, which had weak binding to H929 and did not bind to U266, the other antibodies had strong binding to H929 and U266 cells, which was stronger than or equivalent to the positive control antibody.

**Table 19 FACS detection of binding activity of recombinant candidate antibodies with H929 and U266 cells**

| **Designation of antibody** | **H929** | | **U266** | |
|---|---|---|---|---|
| | **Maximum mean fluorescence intensity** | **EC50 (nM)** | **Maximum mean fluorescence intensity** | **EC50 (nM)** |
| BCMA-Lab09 | 704 | 44.08 | 141 | Negative |
| BCMA-Lab10 | 5154 | 2.91 | 1246 | 1.41 |
| BCMA-Lab11 | 5096 | 4.03 | 1361 | 2.03 |
| BCMA-Lab12 | 4572 | 9.36 | 858 | 4.29 |
| BCMA-Lab13 | 5507 | 3.53 | 1596 | 2.24 |
| BCMA-Lab14 | 4955 | 5.60 | 1299 | 3.50 |
| BCMA-Lab15 | 4648 | 4.12 | 1337 | 2.03 |
| BCMA-Lab16 | 5003 | 3.51 | 1401 | 1.48 |
| BCMA-Lab17 | 3313 | 1.74 | 1010 | 0.98 |
| BCMA-Lab18 | 5272 | 2.79 | 1415 | 1.76 |
| HPN217-hHcAb | 5021 | 4.12 | 1260 | 2.80 |
| hIgG1 | 93 | Negative | 149 | Negative |

### (E) Detection of blocking effect of recombinant candidate antibodies on the binding of ligand APRIL to human BCMA protein by ligand binding competition ELISA

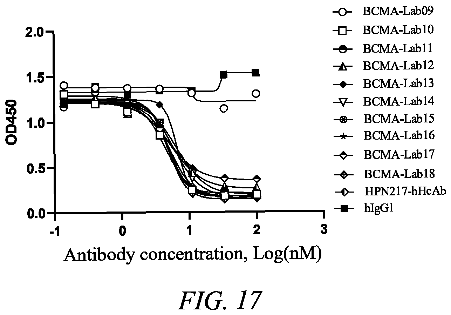

According to the method in Example 4(C), ligand competition ELISA was used to detect the blocking effect of the recombinant antibodies on the binding of the ligand APRIL to human BCMA protein. The results are shown in Fig. 17. The results show that except for BCMA-Lab09, which cannot block the binding of the ligand APRIL to human BCMA protein, the other antibodies could well block the binding of the ligand APRIL to human BCMA protein, and were equivalent to the positive control antibody.

### Example 11 Detection of affinity of BCMA recombinant candidate antibodies

According to the method in Example 5, Biacore was used to detect the affinity of the recombinant antibodies to human BCMA protein. The results are shown in Table 20. The results show that all antibodies had strong affinity to human BCMA protein. The sequences of the nanobodies are shown in Table 21, and the CDR sequences of the nanobodies were shown in Table 22.

**Table 20 Binding affinity of chimeric antibodies to human BCMA protein**

| **Designation of antibody** | **ka (1/Ms)** | **kd (1/s)** | **KD (M)** |
|---|---|---|---|
| BCMA-Lab09 | 5.00E + 04 | 3.99E-03 | 7.97E-08 |
| BCMA-Lab10 | 8.02E + 06 | 5.51E-03 | 6.87E-10 |
| BCMA-Lab11 | 5.83E + 06 | 1.26E-03 | 2.17E-10 |
| BCMA-Lab12 | 6.92E + 05 | 1.03E-02 | 1.49E-08 |
| BCMA-Lab13 | 5.78E + 06 | 1.71E-04 | 2.97E-11 |
| BCMA-Lab14 | 4.43E + 06 | 2.48E-03 | 5.60E-10 |
| BCMA-Lab15 | 2.47E + 06 | 2.19E-03 | 8.87E-10 |
| BCMA-Lab16 | 2.82E + 06 | 1.57E-03 | 5.58E-10 |
| BCMA-Lab17 | 4.15E + 06 | 1.76E-02 | 4.25E-09 |
| BCMA-Lab18 | 2.04E + 06 | 2.06E-03 | 1.01E-09 |
| HPN217-hHcAb | 2.64E + 06 | 3.10E-03 | 1.17E-09 |
| REGN5459-hIgG1 | 7.39E + 05 | 1.97E-04 | 2.67E-10 |

**Table 21 List of sequences of BCMA recombinant nanobodies**

| **Design ation of antibo dy** | **No.** | **Amino acid sequence** |
|---|---|---|
| BCMA -Lab09 | SEQ ID NO: 92 | EVQVVESGGGLVQPGGSLRLSCAASGFTFSSSAMSWARQAPGKGL EWVSGIYPDGSAEYTDSMKGRFTITRDNAKNTVYLQMNSLKPEDT AVYYCAIGRGPSIQAMFRPGGWGQGTQVTVSS |
| BCMA -Lab 10 | SEQ ID NO: 93 | EVQVVESGGGLVQPGGSLRLSCAASGFTFSSSAMSWARQAPGKGR EWVSGIYPDGSTEYADSMKGRFTITRDNAQNTVYLQMDNLRPEDT AVYYCAVGRPINADRPSELWGRGTQVTVSS |
| BCMA -Lab 11 | SEQ ID NO: 94 | QVQLVESGGGLVQPGGSLRLSCAASGSIFSIYAMGWYRQAPGKQR ELVAAITSGTNTYYRDSVKGRFTISRDNAKNTVYLQMNSLKPEDTA VYSCNVAPWGDYDGAADFVSWGQGTQVTVSS |
| BCMA -Lab 12 | SEQ ID NO: 95 | QLQLVESGGGLVQAGGSLRLSCEASGSIFSINAIGWFRQAPGKERE GVSCISRSDGKTYYADSVKGRFTISRDNAKNTVYLQMNSLKPEDT AVYYCATDPSTRYGCYDPALFDSWGQGTQVTVSS |
| BCMA -Lab 13 | SEQ ID NO: 96 | QVQLVESGGGLVQPGGSLRLSCAASGSISSIYAMGWYRQAPGKQR ELVAAIRSAGSTFYRDSVKGRFTISRDNVKNTVYLQMNSLKPEDTA VYQCNVAPWGDYDGAADFTSWGQGTQVTVSS |
| BCMA -Lab 14 | SEQ ID NO: 97 | EVQVVESGGGLVQPGGSLRLSCAASGSIFNIYAMGWYRQAPGKQR ELVAAITSGGSTYYRESVKGRFTISRDNAKNTMYLQMNSLKPEDTA VYYCNVAPWGDYDGAADFDSWGQGTQVTVSS |
| BCMA -Lab 15 | SEQ ID NO: 98 | QLQLVESGGGLVQPGGSLRLSCAGSGFTLDYYMIGWFRQAPGKER EGLSCISPADGSTYYADSVKGRFTISRDSAKNTVYLQMNSLKPEDT ALYYCAAGNEGGISWGFGPYEYDYWGQGTQVTVSS |
| BCMA -Lab 16 | SEQ ID NO: 99 | QLQLVESGGGLVQPGESLRLSCTGSGFTLAYYMIGWFRQAPGKER EGLSCISPADGSTYYADSVKGRFTISRDNAKNVVYLQMNNLKPED TGVYYCAAGNEAAITWGFGPYEYDQWGQGTQVTVSS |
| BCMA -Lab 17 | SEQ ID NO: 100 | QVQLVESGGGLVQPGGSLRLSCTGSGFTLDYYMIGWFRQAPGKER EGLSCISSGDGSTYYADSVKGRFTISRDNAKNTVYLQMNSLKPEDT AVYYCAAGNEATITWGFGPWEYDYWGQGTQVTVSS |
| BCMA -Lab 18 | SEQ ID NO: 101 | QVQLVESGGRLVQPGESLRLSCVASGFTFSSSAMSWARQAPGKGLE WMSGIYPDGTPVYADSMKGRFTVSRDNAKNMVYLQMNSLKPED TAVYYCAVGIRLSDYQGQRRQLLGQGTQVTVSS |

**Table 22 Analysis of CDR sequences by Kabat, IMGT and Chothia numbering**

| **Desi gnat ion of anti bod y** | **Analy sis meth od** | **CDR1** | **CDR2** | **CDR3** |
|---|---|---|---|---|
| BC MA-Lab 09 | Kabat | SSAMS (SEQ ID NO: 71) | GIYPDGSAEYTDSMKG (SEQ ID NO: 102) | GRGPSIQAMFRPGG (SEQ ID NO: 103) |
| | IMGT | GFTFSSSA (SEQ ID NO: 104) | IYPDGSA (SEQ ID NO: 105) | AIGRGPSIQAMFRPG G (SEQ ID NO: 106) |
| | Choth ia | GFTFSSS (SEQ ID NO: 107) | YPDGS (SEQ ID NO: 108) | GRGPSIQAMFRPGG (SEQ ID NO: 103) |
| BC MA-Lab 10 | Kabat | SSAMS (SEQ ID NO: 71) | GIYPD GSTEYAD SMKG (SEQ ID NO: 109) | GRPINADRPSEL (SEQ ID NO: 110) |
| | IMGT | GFTFSSSA (SEQ ID NO: 104) | IYPDGST (SEQ ID NO: 111) | AVGRPINADRPSEL (SEQ ID NO: 112) |
| | Choth ia | GFTFSSS (SEQ ID NO: 107) | YPDGS (SEQ ID NO: 108) | GRPINADRPSEL (SEQ ID NO: 110) |
| BC MA-Lab 11 | Kabat | IYAMG (SEQ ID NO: 113) | AITSGTNTYYRDSVKG (SEQ ID NO: 114) | APWGDYDGAADFVS (SEQ ID NO: 115) |
| | IMGT | GSIFSIYA (SEQ ID NO: 116) | ITSGTNT (SEQ ID NO: 117) | NVAPWGDYDGAAD FVS (SEQ ID NO: 118) |
| | Choth ia | GSIFSIY (SEQ ID NO: 119) | TSGTN (SEQ ID NO: 120) | APWGDYDGAADFVS (SEQ ID NO: 115) |
| BC MA-Lab 12 | Kabat | INAIG (SEQ ID NO: 121) | CISRSDGKTYYADSVK G (SEQ ID NO: 122) | DPSTRYGCYDPALF DS (SEQ ID NO: 123) |
| | IMGT | GSIFSINA (SEQ ID NO: 124) | ISRSDGKT (SEQ ID NO: 125) | ATDPSTRYGCYDPA LFDS (SEQ ID NO: 126) |
| | Choth ia | GSIFSIN (SEQ ID NO: 127) | SRSDGK (SEQ ID NO: 128) | DPSTRYGCYDPALF DS (SEQ ID NO: 123) |
| BC MA-Lab 13 | Kabat | IYAMG (SEQ ID NO: 113) | AIRSAGSTFYRDSVKG (SEQ ID NO: 129) | APWGDYDGAADFTS (SEQ ID NO: 130) |
| | IMGT | GSISSIYA (SEQ ID NO: 131) | IRSAGST (SEQ ID NO: 132) | NVAPWGDYDGAAD FTS (SEQ ID NO: 133) |
| | Choth ia | GSISSIY (SEQ ID NO: 134) | RSAGS (SEQ ID NO: 135) | APWGDYDGAADFTS (SEQ ID NO: 130) |
| BC MA-Lab 14 | Kabat | IYAMG (SEQ ID NO: 113) | AITSGGSTYYRESVKG (SEQ ID NO: 136) | APWGDYDGAADFDS (SEQ ID NO: 137) |
| | IMGT | GSIFNIYA (SEQ ID NO: 138) | ITSGGST (SEQ ID NO: 139) | NVAPWGDYDGAAD FDS (SEQ ID NO: 140) |
| | Choth ia | GSIFNIY (SEQ ID NO: 141) | TSGGS (SEQ ID NO: 142) | APWGDYDGAADFDS (SEQ ID NO: 137) |
| BC MA- | Kabat | YYMIG (SEQ ID NO: 34) | CISPADGSTYYADSVK G (SEQ ID NO: 143) | GNEGGISWGFGPYE YDY (SEQ ID NO: 144) |
| Lab 15 | IMGT | GFTLDYYM (SEQ ID NO: 37) | ISPADGST (SEQ ID NO: 38) | AAGNEGGISWGFGP YEYDY (SEQ ID NO: 145) |
| | Choth ia | GFTLDYY (SEQ ID NO: 40) | SPADGS (SEQ ID NO: 41) | GNEGGISWGFGPYE YDY (SEQ ID NO: 144) |
| BC MA-Lab 16 | Kabat | YYMIG (SEQ ID NO: 34) | CISPADGSTYYADSVK G (SEQ ID NO: 143) | GNEAAITWGFGPYE YDQ (SEQ ID NO: 146) |
| | IMGT | GFTLAYYM (SEQ ID NO: 56) | ISPADGST (SEQ ID NO: 38) | AAGNEAAITWGFGP YEYDQ (SEQ ID NO: 147) |
| | Choth ia | GFTLAYY (SEQ ID NO: 58) | SPADGS (SEQ ID NO: 41) | GNEAAITWGFGPYE YDQ (SEQ ID NO: 146) |
| BC MA-Lab 17 | Kabat | YYMIG (SEQ ID NO: 34) | CISSGDGSTYYADSVK G (SEQ ID NO: 148) | GNEATITWGFGPWE YDY (SEQ ID NO: 149) |
| | IMGT | GFTLDYYM (SEQ ID NO: 37) | ISSGDGST (SEQ ID NO: 150) | AAGNEATITWGFGP WEYDY (SEQ ID NO: 151) |
| | Choth ia | GFTLDYY (SEQ ID NO: 40) | SSGDGS (SEQ ID NO: 152) | GNEATITWGFGPWE YDY (SEQ ID NO: 149) |
| BC MA-Lab 18 | Kabat | SSAMS (SEQ ID NO: 71) | GIYPDGTPVYADSMKG (SEQ ID NO: 153) | GIRLSDYQGQRRQL (SEQ ID NO: 154) |
| | IMGT | GFTFSSSA (SEQ ID NO: 104) | IYPDGTP (SEQ ID NO: 155) | AVGIRLSDYQGQRR QL (SEQ ID NO: 156) |
| | Choth ia | GFTFSSS (SEQ ID NO: 107) | YPDGT (SEQ ID NO: 157) | GIRLSDYQGQRRQL (SEQ ID NO: 154) |

## Claims

1. An antibody or an antigen-binding fragment thereof that specifically binds to BCMA, **characterized in that** the antibody or the antigen-binding fragment thereof comprises CDR1, CDR2 and CDR3, and the CDR1 comprises HCDR1 of a VHH domain as shown in any one of SEQ ID NOs: 7-21, 24-31 and 92-101, the CDR2 comprises HCDR2 of a VHH domain as shown in any one of SEQ ID NOs: 7-21, 24-31 and 92-101, and the CDR3 comprises HCDR3 of a VHH domain as shown in any one of SEQ ID NOs: 7-21, 24-31 and 92-101.

2. The antibody or the antigen-binding fragment thereof of claim 1, **characterized in that** the HCDR1, HCDR2 and HCDR3 are determined according to the Kabat, IMGT or Chothia numbering system;
optionally, the HCDR1 comprises an amino acid sequence as represented by SEQ ID NO: 34, 37, 40, 42, 45, 48, 50, 52, 54, 56, 58, 64, 67, 71, 74, 77, 81, 84, 86, 87, 104, 107, 113, 116, 119, 121, 124, 127, 131, 134, 138 or 141;
optionally, the HCDR2 comprises an amino acid sequence as represented by SEQ ID NOs: 35, 38, 41, 43, 46, 49, 59, 61, 63, 65, 68, 70, 72, 75, 78, 79, 82, 85, 88, 89, 90, 91, 102, 105, 108, 109, 111, 114, 117, 120, 122, 125, 128, 129, 132, 135, 136, 139, 142, 143, 148, 150, 152, 153, 155 or 157;
optionally, the HCDR3 comprises an amino acid sequence as represented by SEQ ID NO: 36, 39, 44, 47, 51, 53, 55, 57, 60, 62, 66, 69, 73, 76, 80, 83, 103, 106, 110, 112, 115, 118, 123, 126, 130, 133, 137, 140, 144, 145, 146, 147, 149, 151, 154 or 156;
preferably, the HCDR1, HCDR2 and HCDR3 comprise amino acid sequences as represented by SEQ ID NOs: 34, 35 and 36; SEQ ID NOs: 37, 38 and 39; or SEQ ID NOs: 40, 41 and 36, respectively;
preferably, the HCDR1, HCDR2 and HCDR3 comprise amino acid sequences as represented by SEQ ID NOs: 42, 43 and 44; SEQ ID NOs: 45, 46 and 47; or SEQ ID NOs: 48, 49 and 44, respectively;
preferably, the HCDR1, HCDR2 and HCDR3 comprise amino acid sequences as represented by SEQ ID NOs: 50, 35 and 51; SEQ ID NOs: 52, 38 and 53; or SEQ ID NOs: 54, 41 and 51, respectively;
preferably, the HCDR1, HCDR2 and HCDR3 comprise amino acid sequences as represented by SEQ ID NOs: 34, 35 and 55; SEQ ID NOs: 56, 38 and 57; or SEQ ID NOs: 58, 41 and 55, respectively;
preferably, the HCDR1, HCDR2 and HCDR3 comprise amino acid sequences as represented by SEQ ID NOs: 34, 59 and 60; SEQ ID NOs: 56, 61 and 62; or SEQ ID NOs: 58, 63 and 60, respectively;
preferably, the HCDR1, HCDR2 and HCDR3 comprise amino acid sequences as represented by SEQ ID NOs: 64, 65 and 66; SEQ ID NOs: 67, 68 and 69; or SEQ ID NOs: 58, 70 and 66, respectively;
preferably, the HCDR1, HCDR2 and HCDR3 comprise amino acid sequences as represented by SEQ ID NOs: 71, 72 and 73; SEQ ID NOs: 74, 75 and 76; or SEQ ID NOs: 77, 78 and 73, respectively;
preferably, the HCDR1, HCDR2 and HCDR3 comprise amino acid sequences as represented by SEQ ID NOs: 34, 79 and 80; SEQ ID NOs: 81, 82 and 83; or SEQ ID NOs: 84, 85 and 80, respectively;
preferably, the HCDR1, HCDR2 and HCDR3 comprise amino acid sequences as represented by SEQ ID NOs: 86, 35 and 36 respectively;
preferably, the HCDR1, HCDR2 and HCDR3 comprise amino acid sequences as represented by SEQ ID NOs: 87, 88 and 44 respectively;
preferably, the HCDR1, HCDR2 and HCDR3 comprise amino acid sequences as represented by SEQ ID NOs: 42, 88 and 44 respectively;
preferably, the HCDR1, HCDR2 and HCDR3 comprise amino acid sequences as represented by SEQ ID NOs: 42, 89 and 44 respectively;
preferably, the HCDR1, HCDR2 and HCDR3 comprise amino acid sequences as represented by SEQ ID NOs: 42, 90 and 44 respectively;
preferably, the HCDR1, HCDR2 and HCDR3 comprise amino acid sequences as represented by SEQ ID NOs: 42, 91 and 44 respectively;
preferably, the HCDR1, HCDR2 and HCDR3 comprise amino acid sequences as represented by SEQ ID NOs: 71, 102 and 103; SEQ ID NOs: 104, 105 and 106; or SEQ ID NOs: 107, 108 and 103, respectively;
preferably, the HCDR1, HCDR2 and HCDR3 comprise amino acid sequences as represented by SEQ ID NOs: 71, 109 and 110; SEQ ID NOs: 104, 111 and 112; or SEQ ID NOs: 107, 108 and 110, respectively;
preferably, the HCDR1, HCDR2 and HCDR3 comprise amino acid sequences as represented by SEQ ID NOs: 113, 114 and 115; SEQ ID NOs: 116, 117 and 118; or SEQ ID NOs: 119, 120 and 115, respectively;
preferably, the HCDR1, HCDR2 and HCDR3 comprise amino acid sequences as represented by SEQ ID NOs: 121, 122 and 123; SEQ ID NOs: 124, 125 and 126; or SEQ ID NOs: 127, 128 and 123, respectively;
preferably, the HCDR1, HCDR2 and HCDR3 comprise amino acid sequences as represented by SEQ ID NOs: 113, 129 and 130; SEQ ID NOs: 131, 132 and 133; or SEQ ID NOs: 134, 135 and 130, respectively;
preferably, the HCDR1, HCDR2 and HCDR3 comprise amino acid sequences as represented by SEQ ID NOs: 113, 136 and 137; SEQ ID NOs: 138, 139 and 140; or SEQ ID NOs: 141, 142 and 137, respectively;
preferably, the HCDR1, HCDR2 and HCDR3 comprise amino acid sequences as represented by SEQ ID NOs: 34, 143 and 144; SEQ ID NOs: 37, 38 and 145; or SEQ ID NOs: 40, 41 and 144, respectively;
preferably, the HCDR1, HCDR2 and HCDR3 comprise amino acid sequences as represented by SEQ ID NOs: 34, 143 and 146; SEQ ID NOs: 56, 38 and 147; or SEQ ID NOs: 58, 41 and 146, respectively;
preferably, the HCDR1, HCDR2 and HCDR3 comprise amino acid sequences as represented by SEQ ID NOs: 34, 148 and 149; SEQ ID NOs: 37, 150 and 151; or SEQ ID NOs: 40, 152 and 149, respectively;
and preferably, the HCDR1, HCDR2 and HCDR3 comprise amino acid sequences as represented by SEQ ID NOs: 71, 153 and 154; SEQ ID NOs: 104, 155 and 156; or SEQ ID NOs: 107, 157 and 154, respectively.

3. The antibody or the antigen-binding fragment thereof of claim 1 or 2, **characterized in that** the CDR1, CDR2 and/or CDR3 comprise(s) an amino acid sequence with 1, 2 or 3 mutations on the HCDR1, HCDR2 and/or HCDR3 of claim 1 or 2; the mutations are selected from insertion, deletion and/or substitution, and the substitution is preferably a conservative amino acid substitution.

4. The antibody or the antigen-binding fragment thereof of claim 1 or 2, **characterized in that** the CDR1, CDR2 and/or CDR3 comprise(s) an amino acid sequence having at least 80%, 85%, 90%, 91%, 92%, 93%, 94%, 95%, 96%, 97%, 98%, 99% or 100% identity to the aforementioned HCDR1, HCDR2 and/or HCDR3.

5. The antibody or the antigen-binding fragment thereof of any one of claims 1 to 4, **characterized in that** the antibody or the antigen-binding fragment thereof comprises a single domain antibody, and the single domain antibody comprises the CDR1, CDR2 and CDR3 of any one of claims 1 to 4;
preferably, the single domain antibody comprises an amino acid sequence as shown in any one of SEQ ID NOs: 7-21, 24-31 and 92-101;
optionally, the single domain antibody comprises an amino acid sequence with at most 20, 19, 18, 17, 16, 15, 14, 13, 12, 11, 10, 9, 8, 7, 6, 5, 4, 3, 2 or 1 mutation relative to the amino acid sequence as shown in any one of SEQ ID NOs: 7-21, 24-31 and 92-101, the mutation is selected from insertion, deletion and/or substitution, and the substitution is preferably a conservative amino acid substitution;
optionally, the single domain antibody comprises an amino acid sequence having at least 80%, 85%, 90%, 91%, 92%, 93%, 94%, 95%, 96%, 97%, 98%, 99% or 100% identity to the amino acid sequence as shown in any one of SEQ ID NOs: 7-21, 24-31 and 92-101.

6. The antibody or the antigen-binding fragment thereof of any one of claims 1 to 5, **characterized in that** the antibody comprises an FR region in the VHH domain as shown in any one of SEQ ID NOs: 7-21, 24-31 and 92-101;
optionally, the antibody comprises an amino acid sequence with at most 15, 14, 13, 12, 11, 10, 9, 8, 7, 6, 5, 4, 3, 2 or 1 mutation relative to the FR region in the VHH domain as shown in any one of SEQ ID NOs: 7-21, 24-31 and 92-101, the mutation is selected from insertion, deletion and/or substitution, and the substitution is preferably a conservative amino acid substitution;
optionally, the antibody comprises an amino acid sequence having at least 80%, 85%, 90%, 91%, 92%, 93%, 94%, 95%, 96%, 97%, 98%, 99% or 100% identity to the FR region in the VHH domain as shown in any one of SEQ ID NOs: 7-21, 24-31 and 92-101.

7. The antibody or the antigen-binding fragment thereof of any one of claims 1 to 6, **characterized in that** the antibody or the antigen-binding fragment thereof is selected from: (1) a chimeric antibody or a fragment thereof; (2) a humanized antibody or a fragment thereof; or (3) a fully human antibody or a fragment thereof.

8. The antibody or the antigen-binding fragment thereof of any one of claims 1 to 7, **characterized in that** the antibody or the antigen-binding fragment thereof comprises or does not comprise an antibody heavy chain constant region; optionally, the antibody heavy chain constant region is selected from humans, llamas, mice, rats, rabbits and goats; optionally, the antibody heavy chain constant region is selected from IgG, IgM, IgA, IgE and IgD; optionally, the IgG is selected from IgG1, IgG2, IgG3 and IgG4; optionally, the heavy chain constant region is selected from an Fc region, a CH3 region and a complete heavy chain constant region, preferably, the heavy chain constant region is a human Fc region; and preferably, the antibody or the antigen-binding fragment thereof is a heavy chain antibody.

9. The antibody or the antigen-binding fragment thereof of any one of claims 1 to 8, **characterized in that** the antibody or the antigen-binding fragment thereof is further coupled with a therapeutic agent or a tracer; preferably, the therapeutic agent is selected from a radioisotope, a chemotherapeutic drug and an immunomodulator, and the tracer is selected from a radiological contrast agent, a paramagnetic ion, a metal, a fluorescent tag, a chemiluminescence tag, an ultrasonic contrast agent and a photosensitizer.

10. The antibody or the antigen-binding fragment thereof of any one of claims 1 to 9, **characterized in that** the antibody or the antigen-binding fragment thereof is also linked to other functional molecules, and preferably, the other functional molecules are selected from one or more of the following: a signal peptide, a protein tag, a cytokine, an angiogenesis inhibitor and an immune checkpoint inhibitor.

11. A multispecific antigen-binding molecule, **characterized in that** the multispecific antigen-binding molecule comprises the antibody or the antigen-binding fragment thereof of any one of claims 1 to 10, and an antigen-binding molecule binding to an antigen other than BCMA or binding to a different BCMA epitope than that of the antibody or the antigen-binding fragment thereof of any one of claims 1 to 10; optionally, the antigen other than BCMA is selected from: CD3 (preferably CD3ε), CD16, CD137, CD258, PD-1, PD-L1, 4-1BB, CD40, CD64, EGFR, VEGF, HER2, HER1, HER3, IGF-1R, phosphatidylserine (PS), C-Met, HSA, GPRC5D, MSLN, blood-brain barrier receptor, GPC3, PSMA, CD33, GD2, ROR1, ROR2, FRα and Gucy2C;
preferably, the antigen-binding molecule is an antibody or an antigen-binding fragment thereof;
preferably, the multispecific antigen-binding molecule is bispecific, trispecific or tetraspecific;
preferably, the multispecific antigen-binding molecule is bivalent, trivalent, tetravalent, pentavalent or hexavalent.

12. An isolated nucleic acid fragment, **characterized in that** the nucleic acid fragment encodes the antibody or the antigen-binding fragment thereof of any one of claims 1-10 or the multispecific antigen-binding molecule of claim 11.

13. A vector, **characterized in that** the vector comprises the nucleic acid fragment of claim 12.

14. A host cell, **characterized in that** the host cell comprises the nucleic acid fragment of claim 12 or the vector of claim 13; and preferably, the cell is a prokaryotic cell or a eukaryotic cell, such as a bacterial (for example, *Escherichia coli*) cell, a fungal (for example, yeast) cell, an insect cell or a mammalian cell (for example, a CHO cell line or a 293T cell line).

15. A method for preparing the antibody or the antigen-binding fragment thereof of any one of claims 1 to 10 or the multispecific antigen-binding molecule of claim 11, **characterized in that** the method comprises culturing the cell of claim 14, and isolating the antibody, the antigen-binding fragment or the multispecific antigen-binding molecule expressed by the cell.

16. A pharmaceutical composition, **characterized in that** the pharmaceutical composition comprises the antibody or the antigen-binding fragment thereof of any one of claims 1 to 10, the multispecific antigen-binding molecule of claim 11, the nucleic acid fragment of claim 12, the vector of claim 13 or a product prepared according to the method of claim 15; optionally, the pharmaceutical composition further comprises a pharmaceutically acceptable carrier, a diluent or an auxiliary agent; and optionally, the pharmaceutical composition further comprises an additional anti-tumor agent.

17. A method for treating a tumor or a cancer, **characterized in that** the method comprises administering to a subject an effective amount of the antibody or the antigen-binding fragment thereof of any one of claims 1 to 10, the multispecific antigen-binding molecule of claim 11, the nucleic acid fragment of claim 12, the vector of claim 13, a product prepared according to the method of claim 15 or the pharmaceutical composition of claim 16; optionally, the tumor or the cancer is a BCMA-expressing tumor or cancer, preferably, the tumor or the cancer is B-cell lymphoma; and more preferably multiple myeloma (MM).

18. The antibody or the antigen-binding fragment thereof of any one of claims 1 to 10, the multispecific antigen-binding molecule of claim 11, the nucleic acid fragment of claim 12, the vector of claim 13, a product prepared according to the method of claim 15 or the pharmaceutical composition of claim 16 for use in the preparation of a drug for treating a tumor or a cancer; optionally, the tumor or the cancer is a BCMA-expressing tumor or cancer, preferably, the tumor or the cancer is B-cell lymphoma; and more preferably multiple myeloma (MM).

19. The antibody or the antigen-binding fragment thereof of any one of claims 1 to 10, the multispecific antigen-binding molecule of claim 11, the nucleic acid fragment of claim 12, the vector of claim 13, a product prepared according to the method of claim 15 or the pharmaceutical composition of claim 16 for treating a tumor or a cancer; optionally, the tumor or the cancer is a BCMA-expressing tumor or cancer, preferably, the tumor or the cancer is B-cell lymphoma; and more preferably multiple myeloma (MM).

20. A kit, **characterized in that** the kit comprises the antibody or the antigen-binding fragment thereof of any one of claims 1 to 10, the multispecific antigen-binding molecule of claim 11, the nucleic acid fragment of claim 12, the vector of claim 13, a product prepared according to the method of claim 15 or the pharmaceutical composition of claim 16.

21. A method for detecting BCMA expression in a biological sample, **characterized in that** the method comprises contacting the biological sample with the antibody or the antigen-binding fragment thereof of any one of claims 1 to 10 under conditions that allow the formation of a complex from the antibody or the antigen-binding fragment thereof and BCMA; and preferably, the method further comprises detecting the formation of the complex, thereby indicating the presence or expression level of BCMA in the sample.

22. The antibody or the antigen-binding fragment thereof of any one of claims 1 to 10 for use in the preparation of a reagent for detecting BCMA.
